# EUROPEAN PATENT APPLICATION

(11) **EP 3 900 729 A1**
(43) Date of publication of application: **27.10.2021**
(21) Application number: 19901304.6
(22) Date of filing: 27.09.2019
(51) Int. Cl.: A61K 31/706, A61K 9/20, A61K 31/436, A61K 38/13, A61P 15/00, A61P 37/02, A61P 43/00

(54) **AGENT FOR IMPROVING INFERTILITY, RECURRENT MISCARRIAGE, AND STATE OF PREGNANCY**

(30) Priority: 18.12.2018 JP 2018236145
(71) Applicant: Yamaguchi, Koushi, Koganei-shi, Tokyo 184-0004 (JP)
(72) Inventor: Yamaguchi, Koushi, Koganei-shi, Tokyo 184-0004 (JP)
(74) Representative: Schön, Christoph
(86) International application number: PCT/JP2019/038213
(87) International publication number: WO 2020/129348

(57) **Abstract**

Provided is a medicine for inhibiting over-activated immunity before pregnancy or over-reactive immunity after pregnancy and restoring normal immune state.

A medicine for inhibiting over-activated immunity before pregnancy or over-reactive immunity after pregnancy and restoring a normal immune state, the medicine including, as an active ingredient, a compound selected from the group consisting of: (i) a compound represented by Formula (I) or a pharmaceutically acceptable salt thereof 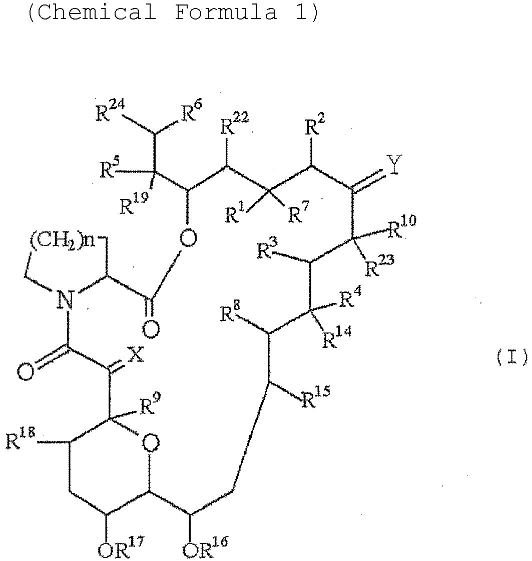 (wherein the respective reference numerals are as described in the specification), (ii) a cyclosporine, and (iii) rapamycin or a derivative thereof.

## Description

### Technical Field

The present invention relates to a medicine for ameliorating sterility and infertility or a pregnancy condition, all of which are affected by immune interactions between the mother's body and the fetus, the medicine including a specific immunosuppressive component. In particular, a representative example of such a medicine may be a medicine for inhibiting over-activated immunity before pregnancy or over-reactive immunity after pregnancy and restoring a normal immune state.

### Background Art

It is estimated that there are about 190,000,000 people worldwide with sterility and infertility, including repeated implantation failure (RIF) and recurrent pregnancy loss (RPL), and this number corresponds to 8% to 12% of couples who can produce children [Reference Literature 1].

There are a large number of causes for sterility and infertility, and the causes are roughly classified into problems with the fertilized ovum and fetus, problems with the mother's body, problems related to an interaction between the mother's body and the fetus, and the like.

The problems with the fertilized ovum or fetus or the problems with the mother's body are treated by means of existing therapeutic methods, and 80% to 90% of the patients can succeed in giving birth to children. However, the remaining 10% to 20% of the patients include sterility and infertility patients due to abnormalities related to immunity between the mother's body and the fetus, the immunity being an interaction between the mother's body and the fetus, and treatment is difficult with the existing therapeutic methods [Reference Literature 2].

It is conceived that one reason for this difficulty lies in the delay of development of novel therapy focused on these abnormalities related to immunity between the mother's body and the fetus.

Several immunosuppressants or immunomodulators have been considered as candidates, and therapeutic methods thereof have been raised as an approach to this problem; however, there is hardly any therapeutic method appropriate and effective for clinical use at the moment [Reference Literature 3].

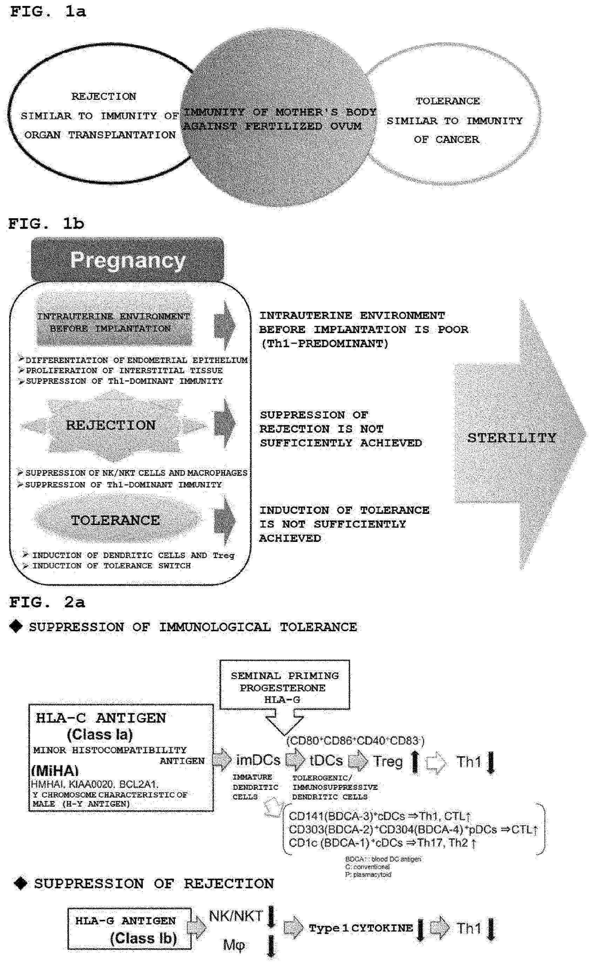

A favorable immune interaction between the mother's body and the fetus mainly inhibits rejection immunity to the fetus and induces immunological tolerance. Immunology in the fields of organ transplantation [Reference Literatures 4 to 12] and cancer [Reference Literatures 13 to 18] is in rapid progress, and many common immune mechanisms are shared by these fields and pregnancy (Fig. 1a).

Natural immunity that rapidly eliminates foreign substances which have invaded the body, and acquired immunity that induces attack on exogenous antigens or immunological tolerance based on the information recognized by dendritic cells (DC), are both fundamental immunological mechanisms; however, these mechanisms exhibit respectively different characteristics in the fields of organ transplantation, cancer, and pregnancy.

Principal immune mechanisms for transplantation include a rejection reaction by T cells induced by direct recognition of a peptide-allogeneic HLA (allo-HLA) complex, which is expressed on the surface of donor dendritic cells, and indirect recognition of an allo-peptide-self-HLA complex, which is expressed on the surface of recipient dendritic cells, the reaction being induced by T cell antigen receptors [Reference Literatures 4 to 7].

It is conceived that a strong graft reaction is mainly caused by direct recognition by T cells, and a graft reaction is associated with chronic rejection caused by anti-allo-HLA-specific antibody produced by B cells. Recipient B cells recognize and bind to a peptide-allogeneic HLA (allo-HLA) complex expressed on the surface of donor dendritic cells, and are induced to differentiate into plasma cells by means of Th2 cells indirectly recognizing an allo-HLA-derived peptide-self-HLA class II complex on the surface of the B cells [Reference Literature 8] .

Two evasion mechanisms have been elucidated for the immunity of cancer. Firstly, immunosuppressive cytokine (TGF-β) produced by cancer cells having genetic abnormalities induces bone marrow-derived suppressor cells and regulatory T cells (Treg) [Reference Literatures 15 and 16].

Secondly, IFN-γ produced by cytotoxic T lymphocytes (CTL) induces expression of a programmed death ligand (PD-L1) on the surface of cancer cells and tumor-infiltrating macrophages. CTL is suppressed as PD-1 receptor binds to PD-L1 [Reference Literatures 15, 17, and 18].

Expression of HLA-G on the surface of villous trophoblast cells is a first stage essential for a successful pregnancy and is the key to achieving evasion from natural immunity and inducing immunological tolerance [Reference Literatures 19 to 26]. However, an imbalance in immunity at the materno-fetal interface and a breakthrough of the placental barrier is capable of inducing a conventional immune response similarly to the responses to other foreign substances and consequently has an adverse effect on placenta construction and embryo development or causes complications in the mother's body. Recognition of fetal antigens that have passed through the placenta or fetal antigens in the placenta induces production of antibodies specific to target proteins in the fetal cells in the mother's body.

Immunological reactions during pregnancy are similar to the immune responses observed in transplantation. However, since it is speculated that a sufficient number of DCs do not exist in the fetus at an early stage of pregnancy, the two differ from each other from the viewpoint that there is a possibility that direct recognition of a peptide/allo-HLA complex may be very weak during pregnancy. Furthermore, the antigenicity of presented antigens is weak, since a fetus has one half of alloantigens. The process of antibody production is the same as that of transplantation.

Therefore, natural immunity is an important immunological reaction during pregnancy, and there is a possibility that subsequent T cell activation by DC may be weaker than that in the case of transplantation. Production of an antibody specific to an antigen may be considered in connection with rhesus monkey D (Rh-D) incompatible pregnancy and fetal hemochromatosis. However, there is a possibility that another fetal antigen containing an allo-HLA-derived peptide may also act as a target, and in patients who have experienced pregnancy and have high Th2 cell ratios, Th2 activation involved in sterility and infertility is anticipated.

In order to accept a fetus and succeed in continuation of pregnancy, several countermeasures toward the maternal immune system are required, including creating an environment for implantation, as well as acceptance of a fertilized ovum, suppression of attacks to the fertilized ovum and the fetus, and immunological tolerance (Fig. 1b) [Reference Literatures 19 to 43].

Functional incompetence of these mechanisms brings about uncontrolled immune responses to the fetus and causes sterility and infertility. Furthermore, there is a possibility that such sterility and infertility may occur not only in women who have no experience in pregnancy or women who substitute for partners, but also even after a miscarriage or delivery.

It is generally conceived that tolerance of the mother's body for the same antigen is enhanced after a success in pregnancy and delivery; however, even implantation failure, miscarriage, and delivery increase the chance for the mother's body to recognize fetal antigens, and as a result, the possibility of rejection reactions is increased. These phenomena may increase sensitization to fetal antigens and promote rejection reactions. There is a possibility that existing therapeutic methods may not achieve successful pregnancies in these patients.

Detailed immune mechanisms acting during normal pregnancies have not been completely elucidated; however, regarding the opportunity of inducing immunological tolerance, immunological tolerance can be induced before or after implantation by including a mother who has previously experienced a pregnancy with the same partner. A rejection reaction occurring due to the mother's natural immunity needs to be suppressed in each occasion of pregnancies.

Fetus-derived extravillous trophoblast does not express histocompatibility antigens HLA-A, HLA-B, or HLA-D [Reference Literature 44], and therefore, the extravillous trophoblast does not become a target of CTL but may become a target of natural killer (NK) cells. However, the trophoblast expresses HLA-C, HLA-E, and HLA-G instead of the above-described HLA types [Reference Literatures 19, 20, and 45 to 49].

Feto-maternal HLA-C mismatch is related to maternal T cell activation and is capable of switching immune response to immunological tolerance under several conditions including seminal priming, sufficient presence of progesterone, and HLA-G [Reference Literatures 26 and 45 to 48] (Fig. 2a). This reaction promotes differentiation of DC from immature DC (imDC) to tolerogenic DC (tDC) and subsequently induces immunological tolerance by means of directed regulatory T cells [Reference Literatures 50 to 53]. Similar findings are also observed with regard to minor histocompatibility antigens such as HMHAI, KIAA0020, BCL2A1, and male antigens (H-Y), and these are expressed on extravillous trophoblast cells [Reference Literatures 54 to 57].

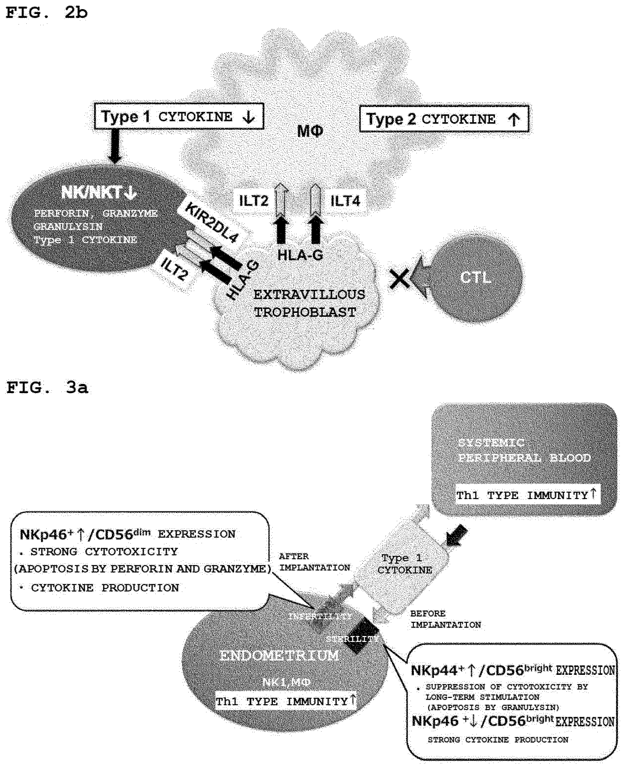

HLA-E suppresses the activity of NK cells in the decidua [Reference Literature 49], and expression of HLA-G on the surface of extravillous trophoblast cells induces evasion from the attack of NK cells and inhibition of macrophage activation [Reference Literatures 21 to 24]. Suppressed NK and NKT cells down-regulate production of perforin and granzyme or granulysin as well as type 1 cytokine. Production of type 1 cytokine from macrophages is also suppressed, and this contributes to reduced enhancement of NK and NKT cell activity (Fig. 2b).

A main mechanism of an immunological rejection reaction in a patient with RIF or RPL is Th1 type immunity [Reference Literatures 58 to 64], and this overlaps with but is not identical with Th1, Th2, Th17, Treg, NK and NKT, and DC in peripheral blood [Reference Literature 63]. Immunological changes occurring in the uterus are as described above; however, usually, immunological problems of RIF patients are raised as a result of Th1 type immunity being dominant, and the activity of Th1 type immunity continuously increases from before pregnancy. On the other hand, RPL patients have various factors that activate immunological rejection reaction against the fetus.

An immune reaction is initiated after implantation, and fetal antigens can be recognized via the following three routes at the materno-fetal interface during pregnancy:
- extravillous trophoblast after implantation,
- syncytiotrophoblast in the placenta, and
- circulation of fetal cells or antigens in the mother's body after completion of the placenta.

A hypothesis that immunological changes differ between RIF and RPL is supported by subsets of NK cells in the uterus of a sterility and infertility patient. It has been reported that in RIF patients, CD56^{bright}/NKp44+ cells increased, and CD56^{dim}/NKp46+ cells decreased, while CD56^{dim}/NKp46+ cells increased in RPL patients [Reference Literatures 37 and 65 to 70] (Fig. 3a).

Other NK cells attack target cells by different mechanisms. CD56^{bright}/NKp44+ and CD56^{dim}/NKp46+ cells together with granzyme B induce target cell apoptosis by means of granulysin and perforin, respectively [Reference Literatures 71 and 72]. Long-term stimulation is needed to induce granulysin production from CD56^{brim}/NKp44+ cells but does not induce perforin that is accompanied by granzyme B production in CD56^{dim}/NKp46+ cells. Therefore, long-term exposure of Th1 type immunity leads to activation of CD56^{bright}/NKp44+ cells in the uterus of an RIF patient [Reference Literatures 71 and 72].

It is relatively easy to identify diagnostic parameters based on these findings and select an RIF patient who can be acclimatized to immunosuppressant treatment. However, in RPL patients, since there is a plurality of opportunities for the mother's body to recognize fetal antigens at different times through different routes, the selection of an acclimatized patient is more complicated.

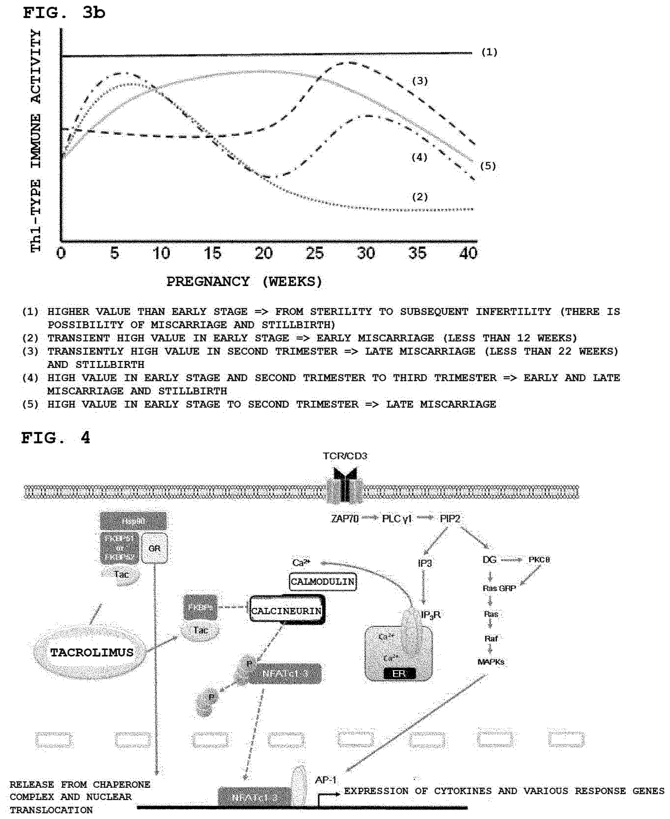

Implantation of a fertilized ovum, infiltration of villi into the decidua in the uterus, and migration of antigens from the fetus to the blood circulation in the mother's body increase with a lapse of time after placental construction and are accelerated after the second trimester of pregnancy. Two peaks of immunological reactions theoretically occur at the materno-fetal interface, and the timing and intensity of these immune reactions against the fetus may vary. Important points to consider in securing safety of the fetus include suppression of alloimmune attack and complete immunological tolerance of the fetus in the early stage of pregnancy. Several patterns of the maternal immune reactions in the uterus are presented in Fig. 3b.

On the other hand, acquired immunity is further classified into "cell-mediated immunity" and "humoral immunity" depending on the type of helper T cells and the way of action. Examples of maternal- and fetal-related diseases or symptoms of the immune system include, for the "cell-mediated immunity", infecundity caused by implantation disorder, infertility caused by placental development disorder, and pregnancy hypertension, and for the maternal- and fetal-related humoral immunity, examples include blood type incompatible pregnancy and fetal hemochromatosis.

With regard to the relationship between immunity and pregnancy, for example, Patent Literature 1 describes a therapeutic drug for sterility and infertility caused by "cell-mediated immunity", the therapeutic drug containing a specific immunosuppressant as an active ingredient.

Placenta is configured to avoid mixing of blood between the mother's body and the fetus; however, a small amount of fetal antigens including blood cells enter the maternal circulation via the placenta (feto-maternal transfusion, FMT). FMT occurs most frequently during childbirth; however, in most cases, there is no clear clinical significance [Reference Literatures 1a to 3a], and FMT can also occur during all pregnancies, including pregnancies that result in triggered or spontaneous abortion.

Antibodies to fetal antigens are produced in the mother's body in a case where the fetal antigens have antigenicity that exceeds the immunological tolerance capacity of the mother's body and are recognized as foreign antigens. The pathogenic antibodies thus produced migrate to the fetal circulation system via the placenta, similarly to other IgG antibodies, and attack target fetal cells or antigens.

After construction of the placenta is completed, the amount of antigens transferred from the fetus to the mother's body increases along with the course of pregnancy. Therefore, the amount of pathogenic antibody production against the fetus in the mother's body also increases, and deterioration of the fetal condition is observed in proportion to the amount of pathogenic antibody production and is accelerated especially after the second trimester of pregnancy.

### Blood type incompatibility

For example, a pregnancy in which the mother's body is Rho (D)-negative while the fetus is Rho (D)-positive is considered to be blood type incompatible pregnancy. After construction of the placenta, a small amount of fetal antigens including blood cell components migrate from the fetus to the mother's body via the placenta. At this time, in a case where a migrated fetal antigen is an antigen exceeding the immunological tolerance capacity of the mother's body, or an antigen that is not tolerated, an antibody is produced by the mother's body, and the antibody migrates to the fetus via the placenta similarly to other antibodies and attacks fetal cells as a target. In blood type incompatible pregnancy, an anti-D antibody produced by the mother's body migrates to the fetus via the placenta, destroys red blood cells in the fetal blood, and causes fetal anemia and later severe hydrops fetalis or intrauterine fetal death [Reference Literature 4a]. This pathologic condition is not prominent in the first pregnancy, and after the second pregnancy, the pathologic condition becomes more severe as every time the chances of sensitization increase. Furthermore, after the placenta is constructed, since the amount of migrated antigens gradually increases along with the course of pregnancy, the pathologic condition progresses with the number of weeks of pregnancy. Particularly, the pathologic condition often deteriorates rapidly after the middle period in which the amount of migration becomes large. A treatment that is currently known is to perform plasmapheresis for removing an anti-D antibody produced by the mother's body, or fetal transfusion for anemia-stricken fetus, and promoting delivery in a state with low risk. Alternatively, in order to prevent sensitization to a D antigen, Rh-incompatible pregnant women must be treated with Rh immunoglobulin (Ig) during pregnancy, and in a case where a patient has not yet been sensitized, the patient should be treated after delivery or abortion. Since this is incompatibility between the mother's body and the fetus not only for RhD but also for other blood types, there is a possibility that blood type incompatible pregnancy may be developed.

A mother having an anti-D antibody needs a management based on Doppler ultrasonography in order to measure the maternal blood antibody titers and the fetal middle cerebral artery (MCA) blood flow velocity, which is a predictive index for fetal anemia during pregnancy [Reference Literature 5a].

On the other hand, fetal hemochromatosis is a disease that causes severe liver failure in the fetal period and the neonatal period, and a cause for the disease is presumed to be allogeneic immune fetal liver disorder. Although no pathogenic antigen has been identified, the disease develops when pregnancy occurs in a situation in which proteins (enzymes and the like) related to fetal iron metabolism are different from those of the mother's body.

After pregnancy, after placenta construction is completed, fetal proteins related to fetal iron metabolism migrate to the mother's body via the placenta, and pathogenic antibodies are produced in the mother's body as a result of maternal immune responses to the migrated fetal protein antigens. Fetal iron metabolism disorder, which is developed when pathogenic antibodies produced in the mother's body migrate to the fetus via the placenta and those antibodies attack fetal proteins related to iron metabolism in the fetal body, is a basic pathogenic condition.

The recurrence rate of fetal hemochromatosis from the same mother's body is 90%, and regarding the treatment, combined therapy of internal medical treatment using iron chelating agents and antioxidants with liver transplantation has been conducted from around 1990; however, the survival rate of the infant was about 50% at the highest. In 2009, a therapeutic method based on postnatal fetal exchange transfusion and large-quantity γ-globulin therapy was reported as a new treatment method, and the survival rate of infants has improved to 75%.

The current treatment method involves subjecting a mother's body during pregnancy to high-dose γ-globulin therapy and preventing the onset of fetal hemochromatosis. However, since a large amount of γ-globulin is required, there is a further demand for the therapeutic method.

### Pregnancy-induced hypertension syndrome

A case where hypertension has been developed during pregnancy is called pregnancy-induced hypertension syndrome (HDP). A case where hypertension is recognized from before pregnancy, or a case where hypertension is recognized up to week 20 of pregnancy is referred to as pregnancy complicated with hypertension (CH); a case where only hypertension is developed after week 20 of pregnancy is classified as gestational hypertension (GH); and a case where hypertension and proteinuria are recognized is classified as preeclampsia (PE). From 2018, although proteinuria is not recognized, when liver function impairment, kidney function impairment, neurological disorder, blood coagulation disorder, and fetal development are defective, this case has been classified as preeclampsia. When pregnancy-induced hypertension syndrome increases in severity, associated diseases such as HELLP syndrome, eclampsia, and central nervous system disorder may be developed.

### HELLP syndrome

HELLP syndrome is a condition presenting a series of symptoms (hemolysis: H, liver function impairment: EL, platelet drop: LP) associated with the risk of life of the mother or the fetus, which occur during pregnancy or during parturition, and is a disease associated with pregnancy-induced hypertension syndrome. When HELLP syndrome is recognized, it is necessary to urgently move to end continuation of pregnancy through precipitous labor or a Caesarean section.

### Eclampsia

Eclampsia is a condition in which a patient develops convulsion, loss of consciousness, or visual field defects together with abnormal hypertension during the perinatal period. Eclampsia may also occur before parturition, during parturition, or in the puerperal period. Eclampsia is circulatory disorders and functional disorders in the brain tissue concomitant with hypertension, and this is also a disease associated with pregnancy-induced hypertension syndrome.

In pregnancy-induced hypertension syndrome and diseases associated therewith (HELLP syndrome, eclampsia, and the like), since both the mother's body and the fetus may be in a very dangerous state, such as fetal growth restriction, premature ablation of normally implanted placenta, fetal functional incompetence, and fetal death, it is a highly important task to prevent the onset or delay the onset of pregnancy-induced hypertension syndrome and diseases associated therewith (eclampsia, HELLP syndrome, and the like) during pregnancy.

The causes for HELLP syndrome and eclampsia are not clearly known; however, it is known that the diseases are complicated with pregnancy-induced hypertension syndrome [Reference Literatures 1b and 2b]. Among the categories of the pregnancy-induced hypertension syndrome, when hypertension, kidney function impairment, aging, and obesity, which are provoking causes as bottom line problems on the mother's side, are excluded, the remaining category is placental construction disorder, and that is considered to be caused by abnormal immunity between the mother's body and the fetus [Reference Literatures 3b to 10b]. The abnormal immunity between the mother's body and the fetus is considered to lack suppression of attack by cell-mediated immunity and humoral immunity by means of T cells, and the immunological tolerance of fetal antigens are insufficient [Reference Literature 11b].

### Citation List

### Patent Literature

Patent Literature 1: WO 2016/068208 A

### Summary of Invention

### Technical Problem

It is an object of the present invention to provide a medicine for ameliorating sterility and infertility or a pregnancy condition, all of which are affected by immune interactions between the mother's body and the fetus, the medicine including a specific immunosuppressive component, particularly a medicine for inhibiting over-activated immunity before pregnancy or over-reactive immunity after pregnancy and restoring a normal immune state.

In addition, it is another object of the present invention to treat or ameliorate blood type incompatible pregnancy or fetal hemochromatosis by restoring a normal immune state.

Furthermore, it is still another object of the present invention to prevent or delay the onset of pregnancy-induced hypertension syndrome and/or diseases associated therewith, by promoting suppression of activation of rejection immunity of the mother's body to the fetus and tolerance to the fetus.

### Solution to Problem

That is, in order to solve the above-described problems, the present invention includes the following embodiments.

### (Embodiment 1)

A medicine for inhibiting over-activated immunity before pregnancy or over-reactive immunity after pregnancy and restoring a normal immune state,
the medicine including, as an active ingredient, a compound selected from the group consisting of:
(i) a compound represented by Formula (I) or a pharmaceutically acceptable salt thereof, 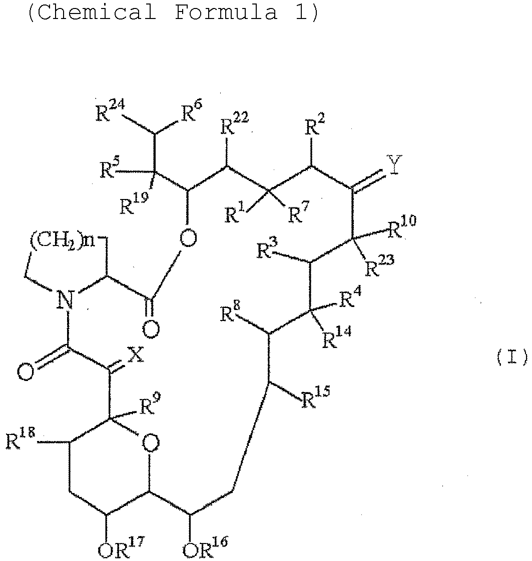 wherein each of the adjacent pairs of R¹ with R², R³ with R⁴, and R⁵ with R⁶ independently
   (a) represents two adjacent hydrogen atoms, or R² may be an alkyl group, or
   (b) may form another bond between the carbon atoms to which the pair members are respectively bonded;

   R⁷ represents a hydrogen atom, a hydroxy group, or a protected hydroxy group, or may be bonded to R¹ and together represent an oxo group;
   R⁸ and R⁹ each independently represent a hydrogen atom or a hydroxy group;
   R¹⁰ represents a hydrogen atom, an alkyl group, an alkyl group substituted with one or more hydroxy groups, an alkenyl group, an alkenyl group substituted with one or more hydroxy groups, or an alkyl group substituted with an oxo group;
   X represents an oxo group, (a hydrogen atom, a hydroxy group), (a hydrogen atom, a hydrogen atom), or a group represented by formula: -CH₂O-;
   Y represents an oxo group, (a hydrogen atom, a hydroxy group), (a hydrogen atom, a hydrogen atom), or a group represented by formula: N-NR¹¹R¹² or formula: N-OR¹³;
   R¹¹ and R¹² each independently represent a hydrogen atom, an alkyl group, an aryl group, or a tosyl group;
   R¹³, R¹⁴, R¹⁵, R¹⁶, R¹⁷, R¹⁸, R¹⁹, R²², and R²³ each independently represent a hydrogen atom or an alkyl group;
   R²⁴ represents a ring which can include one or more heteroatoms and may be substituted as desired;
   n represents 1 or 2; and
   in addition to the meanings described above, Y, R¹⁰, and R²³ may also be bonded together with the carbon atoms to which Y, R¹⁰, and R²³ are bonded, and represent a heterocyclic group having a saturated or unsaturated, 5-membered or 6-membered ring and containing one or more heteroatoms selected from a nitrogen atom, a sulfur atom, and an oxygen atom, while the heterocyclic group may be substituted with one or more groups selected from an alkyl group, a hydroxy group, an alkyloxy group, a benzyl group, a group represented by formula: -CH₂Se(C₆H₅), and an alkyl group substituted with one or more hydroxy groups,
(ii) a cyclosporine, and
(iii) rapamycin or a derivative thereof.

### (Embodiment 2)

A medicine for inducing enhancement of the action of a steroid hormone through activation and nuclear translocation of a steroid hormone receptor,
the medicine including, as an active ingredient, a compound selected from the group consisting of:
(i) a compound represented by Formula (I) or a pharmaceutically acceptable salt thereof,
(ii) a cyclosporine, and
(iii) rapamycin or a derivative thereof.

Here, examples of the steroid hormone include glucocorticoid, mineralocorticoid, estrogen, progesterone, and androgen. With regard to pregnancy, a medicine that makes the intrauterine environment favorable for accepting a fertilized ovum or a fetus through promotion of differentiation of the endometrium and suppression of intrauterine immunity mainly brought by progesterone prior to implantation, can also be provided.

### (Embodiment 3)

A medicine for suppressing the activity of natural killer cells and natural killer T cells or macrophages, which are represented by natural immunity that has a possibility of being activated by a fertilized ovum or a fetal component in the uterus,
the medicine including, as an active ingredient, a compound selected from the group consisting of:
(i) a compound represented by Formula (I) or a pharmaceutically acceptable salt thereof,
(ii) a cyclosporine, and
(iii) rapamycin or a derivative thereof.

### (Embodiment 4)

A medicine for inhibiting the activity of antigen-presenting cells (dendritic cells, macrophages, and the like) presenting antigens of a fertilized ovum or a fetal component, cytotoxic T cells, or T cells producing an intercellular transmitter, all of which have a possibility of directly or indirectly attacking a fertilized ovum or a fetus by acquired immunity in the uterus,
the medicine including, as an active ingredient, a compound selected from the group consisting of:
(i) a compound represented by Formula (I) or a pharmaceutically acceptable salt thereof,
(ii) a cyclosporine, and
(iii) rapamycin or a derivative thereof.

### (Embodiment 5)

A medicine for inducing antigen-presenting cells necessary for subjecting a fertilized ovum or a fetus to immunological tolerance or for inducing differentiation from undifferentiated dendritic cells to tolerant dendritic cells,
the medicine including, as an active ingredient, a compound selected from the group consisting of:
(i) a compound represented by Formula (I) or a pharmaceutically acceptable salt thereof,
(ii) a cyclosporine, and
(iii) rapamycin or a derivative thereof.

### (Embodiment 6)

A medicine for suppressing humoral immunity responses to fetal components including a human leukocyte antigen (HLA), that is, production of a fetus-specific antibody,
the medicine including, as an active ingredient, a compound selected from the group consisting of:
(i) a compound represented by Formula (I) or a pharmaceutically acceptable salt thereof,
(ii) a cyclosporine, and
(iii) rapamycin or a derivative thereof.

Furthermore, pathologic conditions caused by production of a fetus-specific antibody include a fertilized ovum in infecundity or infertility, rejection reactions of a fetus, blood type incompatible pregnancy, neonatal hemochromatosis, and the like.

### (Embodiment 7)

A medicine for suppressing production of a pathogenic antibody in the mother's body, which brings about problems in the continuation of pregnancy,
the medicine including, as an active ingredient, a compound selected from the group consisting of:
(i) a compound represented by Formula (I) or a pharmaceutically acceptable salt thereof,
(ii) a cyclosporine, and
(iii) rapamycin or a derivative thereof. Here, examples of this pathogenic antibody include autoantibodies typified by antiphospholipid antibody syndrome.

### (Embodiment 8)

A medicine for promoting suppression of activation of rejection immunity of the mother's body to the fetus and/or tolerance to the fetus,
the medicine including, as an active ingredient, a compound selected from the group consisting of:
(i) a compound represented by Formula (I) or a pharmaceutically acceptable salt thereof,
(ii) a cyclosporine, and
(iii) rapamycin or a derivative thereof.

### (Embodiment 9)

A medicine for preventing the onset or delay the onset of pregnancy-induced hypertension syndrome and/or a disease associated with pregnancy-induced hypertension syndrome,
the medicine including, as an active ingredient, a compound selected from the group consisting of:
(i) a compound represented by Formula (I) or a pharmaceutically acceptable salt thereof,
(ii) a cyclosporine, and
(iii) rapamycin or a derivative thereof.

### (Embodiment 10)

The medicine according to embodiment 9, wherein the disease associated with pregnancy-induced hypertension syndrome is HELLP syndrome.

### (Embodiment 11)

The medicine according to embodiment 9, wherein the disease associated with pregnancy-induced hypertension syndrome is eclampsia.

### (Embodiment 12)

The medicine according to any one of embodiments 1 to 11, wherein the active ingredient is a compound of Formula (I) or a pharmaceutically acceptable salt thereof, and the compound of Formula (I) is tacrolimus or a pharmaceutically acceptable salt thereof.

Furthermore, the present invention also includes the following embodiments.

### (Embodiment 13)

A medicine for inhibiting over-activated immunity before pregnancy or reaction immunity after pregnancy,
the medicine including, as an active ingredient, a compound selected from the group consisting of:
(i) a compound represented by Formula (I) or a pharmaceutically acceptable salt thereof,
(ii) a cyclosporine, and
(iii) rapamycin or a derivative thereof.

### (Embodiment 14)

The medicine according to embodiment 13, including administering tacrolimus or a pharmaceutically acceptable salt thereof to a patient.

Furthermore, since the compound of the present invention can serve as a medicine for inhibiting over-activated immunity before pregnancy or over-reactive immunity after pregnancy and restoring a normal immune state, it is possible to provide the following treatment using the compound of the present invention, and similarly, the compound of the present invention can serve as a medicine intended for the following treatment.

### (Embodiment 15)

A treatment of a disease associated with humoral immunity in a materno-fetal relationship, or a medicine for the treatment.

### (Embodiment 16)

The treatment according to embodiment 15, or a medicine for the treatment, wherein the disease associated with humoral immunity in a materno-fetal relationship is blood type incompatible pregnancy.

### (Embodiment 17)

The treatment according to embodiment 15, or a medicine for the treatment, wherein the disease associated with humoral immunity in a materno-fetal relationship is fetal hemochromatosis.

### (Embodiment 18)

The treatment according to embodiment 15, or a medicine for the treatment, wherein the treatment is applied to a pregnancy after a second child.

### (Embodiment 19)

The treatment according to embodiment 15, or a medicine for the treatment, wherein the medicine is administered from an early stage of pregnancy.

### (Embodiment 20)

The treatment according to embodiment 15, or a medicine for the treatment, wherein the medicine is administered at a dosage of 1 to 10 mg/day from the early stage of pregnancy.

### (Embodiment 21)

The treatment according to embodiment 15, or a medicine for the treatment, wherein the medicine is administered at a dosage of 3 to 6 mg/day from the early stage of pregnancy.

### (Embodiment 22)

The treatment according to embodiment 15, or a medicine for the treatment, wherein the compound of Formula (I) is administered to a patient having a possibility of blood type incompatible pregnancy in an amount of administration of 1 to 10 mg/day from the early stage of pregnancy.

In addition, when the action mechanism of the compound of the present invention is considered, the compound of the present invention can serve as a medicine for the following method.

### (Embodiment 23)

Suppression of humoral immunity involving the action of a high Th2 cell ratio and activity thereof on B cells.

### Advantageous Effects of Invention

According to the present invention, it is possible to inhibit over-activated immunity before pregnancy or over-reactive immunity after pregnancy, restore a normal immune state, and as a result, treat or ameliorate blood type incompatible pregnancy or fetal hemochromatosis.

Furthermore, according to the present invention, it is possible to promote suppression of activation of rejection immunity of the mother's body to the fetus and tolerance to the fetus, and as a result, prevent or delay the onset of pregnancy-induced hypertension syndrome and/or a disease associated therewith.

### Brief Description of Drawings

Fig. 1a is a diagram showing that maternal immunity shares many common immune mechanisms with organ transplantation and cancer.
Fig. 1b is a diagram showing that acceptance of implantation, suppression of fetal attack, and immunological tolerance of the fetus are important to achieve a success in pregnancy, and there is a possibility that functional incompetence of some of these points may bring about sterility and infertility.
Fig. 2a is a diagram showing that feto-maternal HLA-C mismatch is associated with maternal T cell activation. Immune response can be switched into the direction of immunological tolerance under various conditions.
Fig. 2b is a diagram showing that since extravillous trophoblast (EVT) does not express HLA-A, HLA-B, and HLA-D, the extravillous trophoblast is not a target of cytotoxic T cells, and there is a possibility that it may become a target of NK cells (CD16+/CD56^{dim}). EVT expresses HLA-C, HLA-E, and HLA-G. Expression of HLA-C is related to immunological tolerance, HLA-E suppresses NK cell activity, and HLA-G affects so as to avoid NK cell attack and suppresses production of Type I cytokine by macrophages (see Fig. 3b). ILT denotes Ig-like transcript, KIR denotes kill cell Ig-like receptor, and HLA denotes human leukocyte antigen. HLA-A, HLA-B, and HLA-C belong to MHC class Ia; HLA-E, HLA-F, and HLA-G belong to MHC class Ib; and HLA-DR, HLA-DQ, and HLA-DP belong to MHC class II.
Fig. 3a is a diagram showing that repeated implantation failure (RIF) and recurrent pregnancy loss (RPL) are both associated with predominant Th1-type immunity. Systemically sustained Th1 type-dominant immunity may reflect the condition of uterus before implantation in an RIF patient; however, after implantation in an RPL patient, the condition of uterus may not accurately reflect the condition of the whole body. This theory can be speculated from NK cell subsets in the uterus.
Fig. 3b is an estimation diagram of several variation patterns of Th1-type immunity. The timings of activation are before implantation, immediately after pregnancy when the mother's body recognizes the fetus for the first time, and the second gestation period when fetal antigens enter the maternal circulation through the placenta.
Fig. 4 is a diagram showing the intracellular action mechanism of tacrolimus. Tacrolimus recognizes and binds to a specific receptor and acts on several cellular and molecular pathways. Activated FKBP repressively affects the NFAT pathway through inhibition of calcineurin activity, and by binding to a steroid hormone-chaperon complex, activated FKBP induces release of a hormone receptor from the complex and nuclear translocation thereof. GR denotes a glucocorticoid receptor, NFAT denotes a nuclear factor of an activated T cell, and ER denotes endoplasmic reticulum.

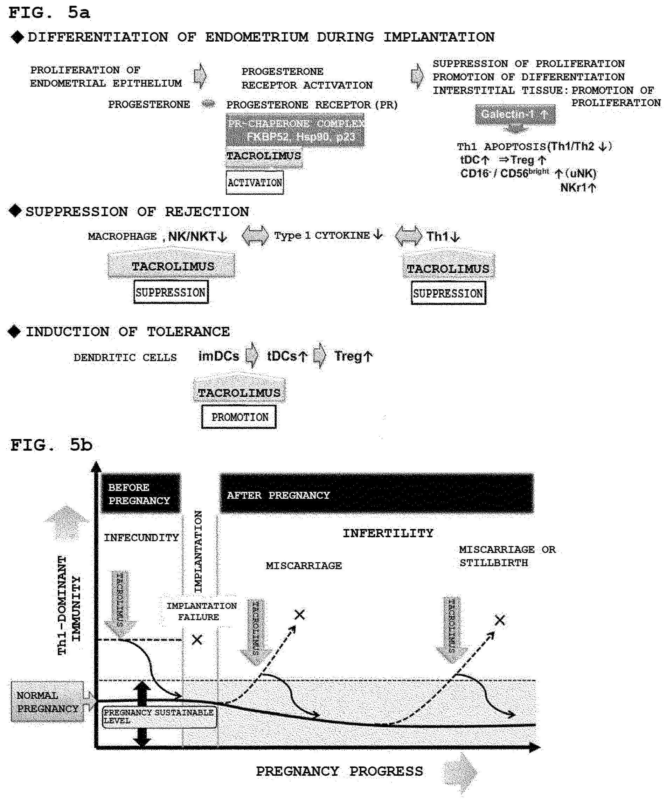

Fig. 5a is a diagram suggesting a possibility of treatment using tacrolimus in sterility and infertility patients. Activation of a progesterone receptor can induce maturation of endometrium during implantation. A main action of tacrolimus is direct suppression of activated NK and NKT cells and activated Th1 cells. Maturation of imDC into tDC is induced, and this may affect immunological tolerance to the fetus.
Fig. 5b is a diagram showing immunological control by tacrolimus for the treatment of sterility and infertility. Tacrolimus is used to restore the level of immunity increased in sterility and infertility to a normal level, unlike the use application in organ transplantation or collagen disease. Therefore, the dose of tacrolimus used for treating sterility and infertility may be a low dose compared to the amount used for other diseases.

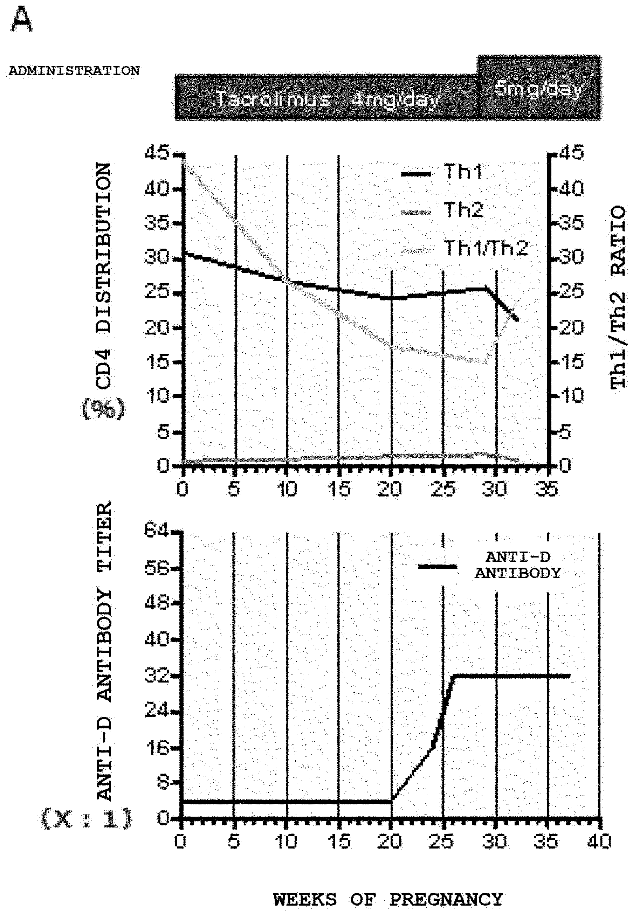

Fig. 6 shows, in the upper part, a graph showing Th1 and Th2 of a patient, and a ratio of the values. The initially high ratio of Th1/Th2 suggests that the patient suffers from infecundity caused by abnormality of the immune system. Fig. 1 shows, in the lower part, a graph showing the titer of an anti-D antibody of a pregnant woman for the weeks of pregnancy. It can be seen that the increase in the antibody titer caused by administration of tacrolimus in the early stage of pregnancy was gentle; however, when the amount of administration was increased (5 mg/day) in week 28 of pregnancy, where the antibody titer started to rapidly increase, the titer of the anti-D antibody did not further increase and was stabilized.

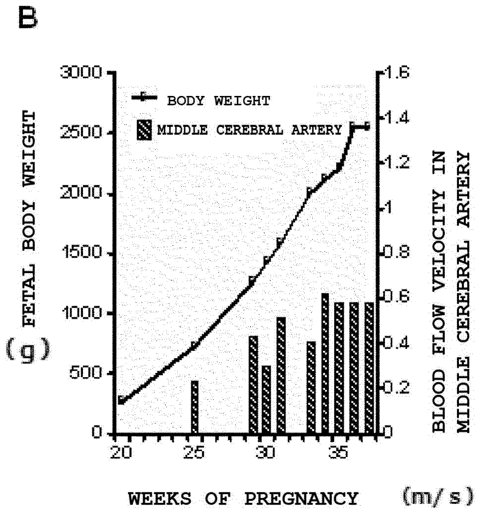

Fig. 7 is a graph showing the body weight of the fetus and the blood flow velocity in the middle cerebral artery in the weeks of pregnancy. The body weight smoothly increased from the early stage of pregnancy until even after week 28 where an increase in the administration of tacrolimus (5 mg/day) was required, the blood flow velocity serving as a measure for fetal anemia corresponded to the number of weeks.

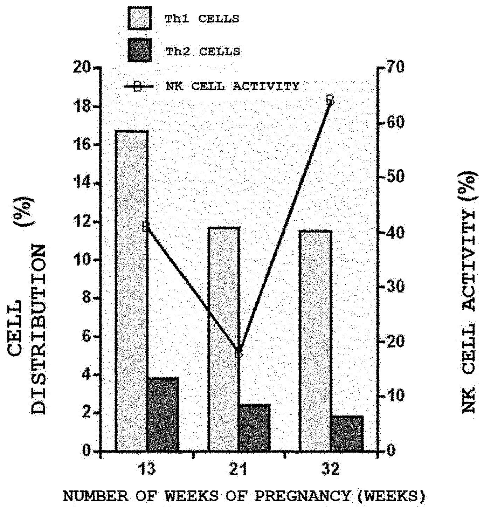

Fig. 8 is a graph showing changes in the proportions of Th1 and Th2 cells among CD4-positive cells and the NK cell activity during pregnancy. While the proportions of Th1 and Th2 cells among CD4-positive cells were continuously decreased, the NK cell activity decreased in the second trimester of pregnancy and then conspicuously increased in the examination in week 32 of pregnancy.

### Description of Embodiments

Hereinafter, the present invention will be described in detail.

That is, the present invention provides a medicine for inhibiting over-activated immunity before pregnancy or over-reactive immunity after pregnancy and restoring a normal immune state,

the medicine including, as an active ingredient, a compound selected from the group consisting of:
(i) a compound represented by Formula (I) or a pharmaceutically acceptable salt thereof, 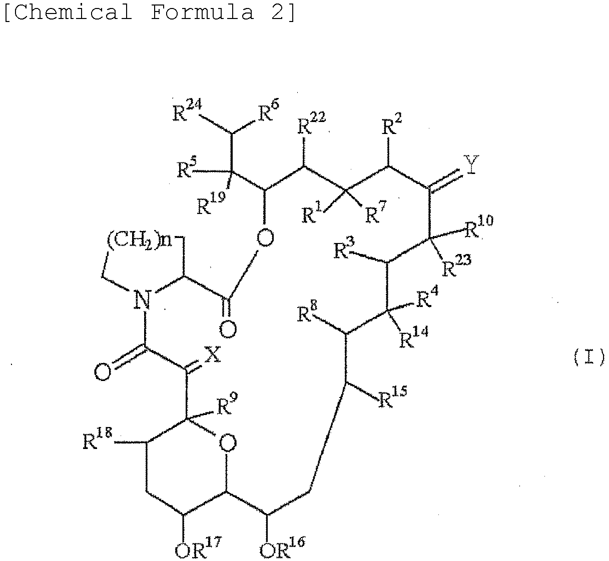 wherein each of the adjacent pairs of R¹ with R², R³ with R⁴, and R⁵ with R⁶ independently
   (a) represents two adjacent hydrogen atoms, or R² may be an alkyl group, or
   (b) may form another bond between the carbon atoms to which the pair members are respectively bonded;

   R⁷ represents a hydrogen atom, a hydroxy group, or a protected hydroxy group, or may be bonded to R¹ and together represent an oxo group;
   R⁸ and R⁹ each independently represent a hydrogen atom or a hydroxy group;
   R¹⁰ represents a hydrogen atom, an alkyl group, an alkyl group substituted with one or more hydroxy groups, an alkenyl group, an alkenyl group substituted with one or more hydroxy groups, or an alkyl group substituted with an oxo group;
   X represents an oxo group, (a hydrogen atom, a hydroxy group), (a hydrogen atom, a hydrogen atom), or a group represented by formula: -CH₂O-;
   Y represents an oxo group, (a hydrogen atom, a hydroxy group), (a hydrogen atom, a hydrogen atom), or a group represented by formula: N-NR¹¹R¹² or formula: N-OR¹³;
   R¹¹ and R¹² each independently represent a hydrogen atom, an alkyl group, an aryl group, or a tosyl group;
   R¹³, R¹⁴, R¹⁵, R¹⁶, R¹⁷, R¹⁸, R¹⁹, R²², and R²³ each independently represent a hydrogen atom or an alkyl group;
   R²⁴ represents a ring which can include one or more heteroatoms and may be substituted as desired;
   n represents 1 or 2; and
   in addition to the meanings described above, Y, R¹⁰, and R²³ may also be bonded together with the carbon atoms to which Y, R¹⁰, and R²³ are bonded, and represent a heterocyclic group having a saturated or unsaturated, 5-membered or 6-membered ring and containing one or more heteroatoms selected from a nitrogen atom, a sulfur atom, and an oxygen atom, while the heterocyclic group may be substituted with one or more groups selected from an alkyl group, a hydroxy group, an alkyloxy group, a benzyl group, a group represented by formula: -CH₂Se(C₆H₅), and an alkyl group substituted with one or more hydroxy groups,
(ii) a cyclosporine, and
(iii) rapamycin or a derivative thereof.

In the compound of Formula (I), R²⁴ represents a ring that can include one or more heteroatoms and may be substituted as desired, and specifically represents a 5-membered to 7-membered carbocyclic ring or a 5-membered or 6-membered heterocyclic group. An example of the 5-membered or 6-membered heterocyclic group may be a saturated or unsaturated, 5-membered or 6-membered heterocyclic group containing one or more heteroatoms selected from a nitrogen atom, a sulfur atom, and an oxygen atom. A preferred example of R²⁴ may be a cyclo- (C₅-C₇) alkyl group which may have an appropriate substituent, and examples thereof include, for example, the following groups:
(a) a 3,4-dioxo-cyclohexyl group;
(b) a 3-R²⁰-4-R²¹-cyclohexyl group
   [wherein R²⁰ represents a hydroxy, an alkyloxy, an oxo, or OCH₂OCH₂CH₂OCH₃, and R²¹ represents a hydroxy, -OCN, an alkyloxy, a heteroaryloxy which may have an appropriate substituent, -OCH₂OCH₂CH₂OCH₃, a protected hydroxy, a chloro, a bromo, an iodo, an aminooxalyloxy, an azide group, a p-tolyloxythiocarbonyloxy, or R²⁵R²⁶CHCOO- (wherein R²⁵ represents a hydroxy group which may be protected as desired, or a protected amino group; and R²⁶ represents a hydrogen atom or a methyl), or R²⁰ and R²¹ may be bonded together and form an oxygen atom of an epoxide ring (that is, -O-)]; or
(c) a cyclopentyl group, the cyclopentyl group possibly being substituted with a methoxymethyl, a hydroxymethyl protected as desired, an acyloxymethyl (wherein the acyl moiety is a dimethylamino group which may be quaternized as desired, or a carboxyl group which may be esterified as desired), one or more amino and/or hydroxy groups which may be protected, or an aminooxalyloxymethyl, while a preferred example is a 2-formylcyclopentyl group.

Various definitions and specific examples thereof as used in the present specification, and preferred embodiments thereof will be described in detail below.

Unless particularly stated otherwise, the term "lower" is intended to mean a group having 1 to 6 carbon atoms.

A preferred example of the alkyl moiety of the "alkyl group" and the "alkyloxy group" may be a linear or branched aliphatic hydrocarbon residue, and examples include lower alkyl groups having 1 to 6 carbon atoms, such as methyl, ethyl, propyl, isopropyl, butyl, isobutyl, pentyl, neopentyl, and hexyl.

A preferred example of the "alkenyl group" may be a linear or branched aliphatic hydrocarbon residue containing one double bond, and examples include lower alkenyl groups such as vinyl, propenyl (allyl or the like), butenyl, methylpropenyl, pentenyl, and hexenyl.

Preferred examples of the "aryl group" include phenyl, tolyl, xylyl, cumenyl, mesityl, and naphthyl.

Examples of a preferred protective group for the "protected hydroxy group" and the "protected amino" include 1-(lower alkylthio) (lower) alkyl groups such as, for example, a lower alkylthiomethyl group such as methylthiomethyl, ethylthiomethyl, propylthiomethyl, isopropylthiomethyl, butylthiomethyl, isobutylthiomethyl, or hexylthiomethyl, with a more preferred example being a C₁-C₄ alkylthiomethyl group, and the most preferred example being a methylthiomethyl group; trisubstituted silyl groups, for example, a tri-(lower) alkylsilyl such as trimethylsilyl, triethylsilyl, tributylsilyl, tertiary butyldimethylsilyl, or tri-tertiary butylsilyl, and for example, a lower alkyldiarylsilyl such as methyldiphenylsilyl, ethyldiphenylsilyl, propyldiphenylsilyl, or tertiary butyldiphenylsilyl, with more preferred examples being a tri(C₁-C₄) alkylsilyl group and a C₁-C₄ alkyldiphenylsilyl group, and the most preferred examples being a tertiary butyldimethylsilyl group and a tertiary butyldiphenylsilyl group; and acyl groups such as an aliphatic acyl group derived from a carboxylic acid, a sulfonic acid or a carbamic acid, an aromatic acyl group, and an aliphatic acyl group substituted with an aromatic group.

Examples of the aliphatic acyl group include a lower alkanoyl group which may have one or more appropriate substituents such as a carboxyl, for example, formyl, acetyl, propionyl, butyryl, isobutyryl, valeryl, isovaleryl, pivaloyl, hexanoyl, carboxyacetyl, carboxypropionyl, carboxybutyryl, or carboxyhexanoyl; a cyclo-(lower) alkyloxy-(lower) alkanoyl group which may have one or more appropriate substituents such as a lower alkyl, for example, cyclopropyloxyacetyl, cyclobutyloxypropionyl, cycloheptyloxybutyryl, menthyloxyacetyl, menthyloxypropionyl, menthyloxybutyryl, menthyloxypentanoyl, or menthyloxyhexanoyl; a camphorsulfonyl group; and a lower alkylcarbamoyl group having one or more appropriate substituents such as a carboxyl or a protected carboxyl, for example, a carboxy-(lower) alkylcarbamoyl group such as carboxymethylcarbamoyl, carboxyethylcarbamoyl, carboxypropylcarbamoyl, carboxybutylcarbamoyl, carboxypentylcarbamoyl, or carboxyhexylcarbamoyl, or a tri-(lower) alkylsilyl-(lower) alkyloxycarbonyl-(lower) alkylcarbamoyl group such as trimethylsilylmethoxycarbonylethylcarbamoyl, trimethylsilylethoxycarbonylpropylcarbamoyl, triethylsilylethoxycarbonylpropylcarbamoyl, tertiary butyldimethylsilylethoxycarbonylpropylcarbamoyl, or trimethylsilylpropoxycarbonylbutylcarbamoyl group.

Examples of the aromatic acyl group include an aroyl group which may have one or more appropriate substituents such as a nitro, for example, benzoyl, toluoyl, xyloyl, naphthoyl, nitrobenzoyl, dinitrobenzoyl, or nitronaphthoyl; and an arenesulfonyl group which may have one or more appropriate substituents such as a halogen, for example, benzenesulfonyl, toluenesulfonyl, xylenesulfonyl, naphthalenesulfonyl, fluorobenzenesulfonyl, chlorobenzenesulfonyl, bromobenzenesulfonyl, or iodobenzenesulfonyl.

Examples of the aliphatic acyl group substituted with an aromatic group include an aryl-(lower) alkanoyl group which may have one or more appropriate substituents such as a lower alkyloxy or a trihalo-(lower) alkyl, for example, phenylacetyl, phenylpropionyl, phenylbutyryl, 2-trifluoromethyl-2-methoxy-2-phenylacetyl, 2-ethyl-2-trifluoromethyl-2-phenylacetyl, or 2-trifluoromethyl-2-propoxy-2-phenylacetyl.

Among the acyl groups described above, more preferred examples of the acyl group include a C₁-C₄ alkanoyl group which may have a carboxyl, a cyclo-(C₅-C₆) alkyloxy- (C₁-C₄) alkanoyl group having two (C₁-C₄) alkyls in the cycloalkyl moiety, a camphorsulfonyl group, a carboxy-(C₁-C₄) alkylcarbamoyl group, a tri- (C₁-C₄) alkylsilyl- (C₁-C₄) alkyloxycarbonyl-(C₁-C₄) alkylcarbamoyl group, a benzoyl group which may have one or two nitro groups, a benzenesulfonyl group having a halogen, and a phenyl-(C₁-C₄) alkanoyl group having a C₁-C₄ alkyloxytrihalo- (C₁-C₄) alkyl, and among them, most preferred examples include acetyl, carboxypropionyl, menthyloxyacetyl, camphorsulfonyl, benzoyl, nitrobenzoyl, dinitrobenzoyl, iodobenzenesulfonyl, and 2-trifluoromethyl-2-methoxy-2-phenylacetyl.

Examples of the "5-membered to 7-membered carbocyclic ring" include 5-membered to 7-membered cycloalkyl groups or cycloalkenyl groups, and examples thereof include cyclopentyl, cyclohexyl, cycloheptyl, cyclopentenyl, cyclohexenyl, and cycloheptenyl.

Preferred examples of the "heterocyclic group having a saturated or unsaturated, 5-membered or 6-membered ring and containing one or more heteroatoms selected from a nitrogen atom, a sulfur atom, and an oxygen atom" include a pyrrolyl group and a tetrahydrofuryl group.

Examples of the "heteroaryl moiety which may have an appropriate substituent" in the "heteroaryloxy which may have an appropriate substituent" include the moieties listed as examples of group R¹ of the compound represented by the formula shown in EP-A-532,088, and for example, 1-hydroxyethylindol-5-yl is preferred. The disclosure of the patent literature is partially incorporated herein by reference.

### Active ingredient

According to the present invention, (i) a compound represented by Formula (I) or a pharmaceutically acceptable salt thereof, (ii) a cyclosporine, or (iii) rapamycin or a derivative thereof can be used as an active ingredient. Furthermore, two or more kinds of (i) a compound represented by Formula (I), (ii) a cyclosporine, or (iii) rapamycin or a derivative thereof may also be used in combination as the active ingredient. The respective active ingredients will be described below.

### (i) Compound represented by Formula (I)

The compound represented by Formula (I) or a pharmaceutically acceptable salt thereof, which is used for the present invention, is as described above, and specifically, the compound or the salt is described in, for example, EP-A-184162, EP-A-323042, EP-A-423714, EP-A-427680, EP-A-465426, EP-A-480623, EP-A-532088, EP-A-532089, EP-A-569337, EP-A-626385, WO 89/05303, WO 93/05058, WO 96/31514, WO 91/13889, WO 91/19495, and WO 93/5059.

Particularly, compounds called FR900506 (= FK506, tacrolimus), FR900520 (ascomycin), FR900523, and FR900525 are substances produced by the genus Streptomyces, for example, Streptomyces tsukubaensis No. 9993 (depository: National Institute of Bioscience and Human-Technology Agency of Industrial Science and Technology, the Ministry of International Trade and Industry, 1-3, Higashi 1-chome, Tsukuba-shi, Ibaraki-ken, Japan (formerly: Fermentation Research Institute, Agency of Industry Science and Technology, the Ministry of International Trade and Industry), date of deposit: Oct. 5, 1984, deposit number: FERM BP-927) or Streptomyces hygroscopicus subsp. yakushimaensis No. 7238 (depository: National Institute of Bioscience and Human-Technology Agency of Industrial Science and Technology, the Ministry of International Trade and Industry, 1-3, Higashi 1-chome, Tsukuba-shi, Ibaraki-ken, Japan, date of deposit: Jan. 12, 1985, deposit number: FERM BP-928) (EP-A-0184162), and particularly, FK506 (general name: tacrolimus) represented by the following structural formula is a representative compound. 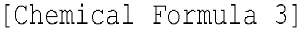 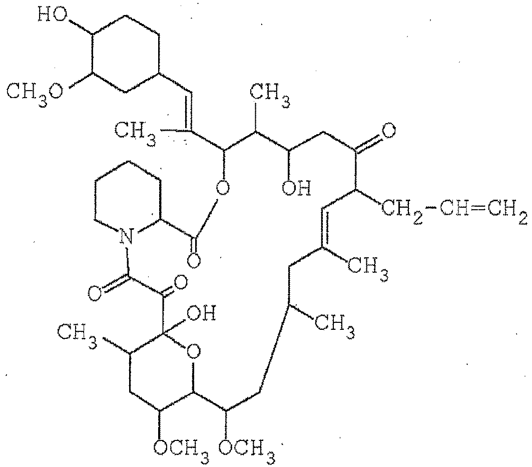 Chemical name: 17-Allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.04,9]octacos-18-ene-2,3,10,16-tetraone.

As a particularly preferred embodiment, the compound represented by Formula (I) is such that each of the adjacent pairs of R³ with R⁴ and R⁵ with R⁶ forms another bond between the carbon atoms to which the pair members are respectively bonded (thus, a double bond is formed at the moieties of R³ with R⁴ and R⁵ with R⁶) ;
R¹, R², R⁸, and R²³ independently represent a hydrogen atom;
R⁹ represents a hydroxy group; R¹⁰ represents a methyl, ethyl, propyl, or allyl group;
R⁷ represents a hydroxy;
X represents (a hydrogen atom, a hydrogen atom) or an oxo group;
Y represents an oxo group;
R¹⁴, R¹⁵, R¹⁶, R¹⁷, R¹⁸, R¹⁹, and R²² each represent a methyl group; and
R²⁴ represents a 3-R²⁰-4-R²¹-cyclohexyl group,
   wherein R²⁰ represents a hydroxy, an alkyloxy, an oxo, or OCH₂OCH₂CH₂OCH₃; and
R²¹ represents a hydroxy, -OCN, an alkyloxy, a heteroaryloxy which may have an appropriate substituent, a 1-tetrazolyl or a 2-tetrazolyl, -OCH₂OCH₂CH₂OCH₃, a protected hydroxy, a chloro, a bromo, an iodo, an aminooxalyloxy, an azide group, a p-tolyloxythiocarbonyloxy, or R²⁵R²⁶CHCOO- (wherein R²⁵ represents a hydroxy group which may be protected as desired, or a protected amino group; and R²⁶ represents a hydrogen atom or a methyl), or
R²⁰ and R²¹ may be bonded together and form an oxygen atom of an epoxide ring (that is, -O-); and n represents 1 or 2.

Other preferred embodiments of the compound represented by Formula (I) include tacrolimus, and ascomycin or a derivative thereof.

Furthermore, the compounds described in EP-0184162, EP 323042, EP 424714, EP 427680, EP 465426, EP 474126, EP 480623, EP 484936, EP 532088, EP 532089, EP 569337, EP 626385, WO 89/05303, WO 93/05058, WO 96/31514, WO 91/13889, WO 91/19495, WO 93/5059, WO 96/31514, and the like may also be listed as preferred examples of the compound represented by Formula (I) of the present invention, the disclosures of which are partially incorporated herein by reference.

### Pharmaceutically acceptable salt of compound represented by Formula (I)

The term "pharmaceutically acceptable salt" for the compound represented by Formula (I) of the present invention refers to a salt prepared from a pharmaceutically acceptable, non-toxic base or acid. In a case where the compound of Formula (I) of the present invention is acidic, a salt corresponding thereto can be conveniently prepared from a pharmaceutically acceptable non-toxic base, which includes an inorganic base and an organic base. Examples of a salt derived from such an inorganic base include salts of aluminum, ammonium, calcium, copper (cupric and cuprous), ferric, ferrous, lithium, magnesium, manganese (manganic and manganous), potassium, sodium, and zinc. Salts of ammonium, calcium, magnesium, potassium, and sodium are preferred. The salt prepared from a pharmaceutically acceptable non-toxic organic base includes salts of primary, secondary, and tertiary amines derived from both naturally occurring and synthetic sources. Examples of a pharmaceutically acceptable non-toxic organic base include arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, dicyclohexylamine, lysine, methylglucamine, morpholine, piperazine, piperidine, a polyamine resin, procaine, purine, theobromine, triethylamine, trimethylamine, tripropylamine, and tromethamine.

In a case where the compound represented by Formula (I) of the present invention is basic, a salt corresponding thereto can be conveniently prepared from a pharmaceutically acceptable non-toxic base, which includes an inorganic acid and an organic acid. Examples of such an acid include acetic acid, benzenesulfonic acid, benzoic acid, camphorsulfonic acid, citric acid, ethanesulfonic acid, fumaric acid, gluconic acid, glutamic acid, hydrobromic acid, hydrochloric acid, isethionic acid, lactic acid, maleic acid, malic acid, mandelic acid, methanesulfonic acid, muconic acid, nitric acid, pamoic acid, pantothenic acid, phosphoric acid, succinic acid, sulfuric acid, tartaric acid, and p-toluenesulfonic acid. Citric acid, hydrobromic acid, hydrochloric acid, maleic acid, phosphoric acid, sulfuric acid, and tartaric acid are preferred.

### Solvate or hydrate of compound represented by Formula (I)

The compound represented by Formula (I) of the present invention may form a solvate, and that case is also included in the scope of the present invention. Preferred examples of the solvate include a hydrate and an ethanolate.

### Crystal form of compound represented by Formula (I)

The compound represented by Formula (I) of the present invention can exist in an amorphous form and/or in one or more crystalline forms, and all such amorphous form and crystalline forms of the compound represented by Formula (I) as well as a mixture of those are intended to be included in the scope of the present invention. Furthermore, the crystalline forms of the compounds represented by Formula (I) can also form solvates with water (that is, hydrates) or solvates with conventional organic solvents. The solvates and hydrates, particularly pharmaceutically acceptable solvates and hydrates, of the crystalline forms of the compounds represented by Formula (I) are likewise included in the scope of the compound defined by Formula (I) and pharmaceutically acceptable salts thereof.

### Isomers of compound represented by Formula (I)

With regard to the compound represented by Formula (I) of the present invention, there may be one or more pairs of steric isomers such as a conformer or an optical isomer attributable to an asymmetric carbon atom and a double bond and a geometrical isomer, and such conformer or isomers are also included in the scope of the compounds of the present invention.

### Method for preparing compound of Formula (I)

The compound of Formula (I) of the present invention is a compound known in the cited references, and a production method for the compound is disclosed in, for example, EP-A-184162, EP-A-323042, EP-A-423714, EP-A-427680, EP-A-465426, EP-A-480623, EP-A-532088, EP-A-532089, EP-A-569337, EP-A-626385, WO 89/05303, WO 93/05058, WO 96/31514, WO 91/13889, WO 91/19495, and WO 93/5059. Furthermore, tacrolimus is marketed from Astellas Pharma, Inc. under the brand name of PROGRAF (registered trademark).

### (ii) Cyclosporine

Examples of the cyclosporine include cyclosporines A, B, and D, and these are described in Merck Index (12th edition) No. 2821. Furthermore, cyclosporines are marketed from Novartis Pharma AG under the brand name of SANDIMMUNE.

### (iii) Rapamycin or derivative thereof

Rapamycin (also called sirolimus) is described in Merck Index (12th edition) No. 8288, and derivatives thereof can also be used. Preferred examples of a rapamycin derivative include O-substituted derivatives in which the hydroxy at the 40-position of Formula (A) in page 1 of WO 95/16691: 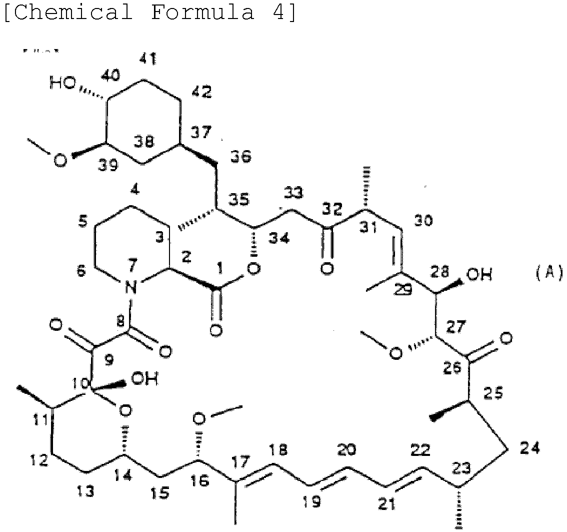 is substituted by -OR¹ (wherein R¹ represents a hydroxyalkyl, a hydroalkyloxyalkyl, an acylaminoalkyl, and an aminoalkyl), for example, 40-O-(2-hydroxy)ethyl-rapamycin, 40-O-(3-hydroxy)propyl-rapamycin, 40-O-[2-(2-hydroxy)ethoxy]ethyl-rapamycin, and 40-O-(2-acetaminoethyl)-rapamycin. Furthermore, rapamycin (sirolimus) is marketed from Nobelpharma Co., Ltd. under the brand name of RAPALIMUS.

The compound represented by Formula (I) of the present invention, a cyclosporine, rapamycin and a derivative thereof have a similar basic skeleton, that is, a tricyclomacrolide skeleton and have at least one similar biological characteristics (for example, immunosuppressive action) .

### Other optional components

The medicine according to the present invention may include, in addition to the above-described active ingredients, one or more therapeutically active substances having therapeutic action on other diseases, illnesses, and symptoms, so long as the therapeutically active substances have no risk of inhibiting the activity of the active ingredient and are not harmful to the subject for administration (hereinafter, also referred to as patient).

Examples of such therapeutically active substances include estrogens including estrone, estradiol, and estriol; progesterone, and prednisolone.

### Medicine of present invention

The medicine of the present invention includes a compound selected from the group consisting of: (i) a compound of Formula (I) or a pharmaceutically acceptable salt thereof, (ii) a cyclosporine, and (iii) rapamycin or a derivative thereof as an active ingredient, and may further include a pharmaceutically acceptable carrier that is not harmful to the subject for administration. The carrier that can be used may be any of a solid type, a semisolid type, and a liquid type and may be, for example, any one selected from water, a liquid electrolyte, a glucose solution, and the like; however, the carrier is not limited to these. Furthermore, the medicine of the present invention may also include auxiliary agents. Examples of the auxiliary agents include a lubricating agent, a stabilizer, an antiseptic agent, an emulsifier, a thickener (viscous agent), a colorant, a fragrance (flavoring agent), an excipient, a preservative, a buffering agent, a corrigent, a suspending agent, an emulsifier, and a dissolution aid.

### Dosage form

The medicine including the active ingredient of the present invention can be provided in various dosage forms, and examples of the dosage forms include a tablet, a capsule, a pill, a granular preparation, a powder, a syrup, a suppository, a troche, a pellet, an emulsion, a suspension, and other known forms. Among these, for example, the dosage form as a preparation for oral administration is preferably any of a tablet, a capsule, a pill, a granular preparation, a powder, a liquid, a syrup, and a jelly; more preferably any of a tablet, a capsule, and a granular preparation; and even more preferably a tablet. In addition, as will be described below, the medicine may be formulated as a preparation for parenteral administration such as, for example, an injectable preparation, a suppository, and a percutaneous absorption type preparation.

### Method for producing medicine

The medicine according to the present invention can be produced by utilizing any known production method. For example, the medicine is produced by producing an active ingredient and other optional components separately for each component, and then mixing the respective components so as to obtain desired contents.

### Subject for administration of medicine

Examples of the subject for administration of the medicine of the present invention include mammals. Examples of the mammals include human being and non-human animals, including domestic animals such as cow, horse, pig, and sheep; monkey, chimpanzee; and pets such as dog, cat, rat, and rabbit, and a preferred mammal is human being.

### Route of administration

The method for administration (route of administration) of the medicine of the present invention can be appropriately determined based on the age and condition of the subject for administration, the duration of treatment, and the like. Specifically, both oral administration and parenteral administration may be employed; however, oral administration is preferred (oral administration is employed in Examples). Examples of parenteral administration include methods such as administration by injection, administration using a suppository, and administration using a percutaneous absorption type preparation. Examples of the type of administration by injection include intramuscular injection, intraperitoneal injection, subcutaneous injection, intravenous injection, and topical injection. Furthermore, the medicine of the present invention can be administered through various routes such as percutaneous, transnasal, transvaginal, and transrectal routes.

### Amount of administration

The amount of administration of the medicine varies depending on the type of the disease, illness, or symptoms of the patient who receives administration of the medicine, severity, results of various examinations, the type of the active ingredient of the medicine, and the like. Furthermore, the amount of administration of the medicine also varies depending on the age of the patient to be treated, the number of times of treatment according to the treatment method of the present invention, results of various examinations, and the like. For instance, from the viewpoint of the content of the active ingredient included in the medicine, the medicine of the present invention is administered at a dose that is lower than the amount of administration in a case where the medicine is used as an immunosuppressant in living donor organ transplantation, the treatment for immune system diseases, and the like. For example, in a case where the subject for administration of the medicine is a human being, without being particularly limited, the medicine is administered in an amount, as an amount of the active ingredient, preferably in the range of 0.5 to 10 mg or 1 to 10 mg, and more preferably in the range of 0.5 to 6 mg or 3 to 6 mg, per day according to the symptoms of the patient. In addition, hereinafter, unless particularly stated otherwise, the description concerning the amount of administration of the medicine is applied in a case where the subject is a human being, and the amount of administration is indicated as the amount of the active ingredient.

Furthermore, without being particularly limited, in the case of oral administration, the frequency of administration per day is preferably 1 to 4 times, more preferably 1 to 3 times, and even more preferably 1 to 2 times.

The medicine including the active ingredient of the present invention can be administered stepwise or in combination with another therapeutic drug. In a case where the medicine is administered in combination with another therapeutic drug, the medicine of the present invention and the other therapeutic drug can be administered as separate preparations, simultaneously or at different intervals of administration.

In a case where the medicine including the active ingredient of the present invention is administered stepwise or in combination with another therapeutic drug, generally the same dosage form can be used. In a case where these drugs are physically combined and administered, the dosage form and the route of administration should be selected according to the compatibility of the drugs to be combined. Therefore, the term simultaneous administration is understood to include simultaneous or continuous administration of two medicines, or administration of two active ingredients as a combination at fixed doses.

Examples of the other therapeutic drug include estrogens including estrone, estradiol, and estriol; progesterone, and prednisolone. Further examples also include immunoglobulins, for example, anti-D immunoglobulin.

Furthermore, the medicine of the present invention may be combined with physical therapy such as plasma exchange therapy and fetal transfusion.

Medicine for inhibiting over-activated immunity before pregnancy or over-reactive immunity after pregnancy and restoring normal immune state

Tacrolimus is one of related substances (tacrolimus, rapamycin, ascomycin, and meridamycin) isolated from soil actinomycetes in Japan. This is a macrolide derivative antibiotic substance having a 23-membered ring macrolide macrolactam structure [Reference Literature 73]. Tacrolimus is a calcium/calmodulin-dependent phosphatase that binds to an FK506-binding protein (FKBP) receptor and inhibits the activity of calcineurin [Reference Literatures 74 to 78]. This calcineurin inhibitor (CNI) inhibits calcium-dependent signals that accomplish a role of transferring nuclear factor of activated T cells (NFAT) to the nucleus in cells stimulated by means of T cell receptors (Fig. 4) [Reference Literatures 79 to 83]. Detailed elucidation of the mechanism of signal transduction in cells is connected to a successful use of tacrolimus in the fields of transplantation and autoimmune diseases [Reference Literatures 84 and 85].

It has been reported that tacrolimus acts not only on the CN-NFAT pathway of T cells but also on other cell types including natural killer (NK) cells and natural killer T (NKT) cells, macrophages, B cells, and dendritic cells (DC cells) [Reference Literatures 86 to 92]. Direct inhibition occurs in T cells, NK and NKT cells, and macrophages. There have been several studies reporting negative effects of tacrolimus on tDC maturation [Reference Literatures 88 and 89], and others disclose a positive role of tacrolimus that induces maturation of immature DC cells (imDC) to tolerogenic DC cells (tDC cells) [Reference Literatures 26, 90, and 91].

B cells (active, antibody-producing, and class-switching) are suppressed by T follicular helper cells 88 [Reference Literature 87]. These findings suggest that tacrolimus can suppress rejection by inhibiting activated NK and NKT cells and macrophages and induce tolerance to the fetus, and can also induce differentiation of imDC to tDC.

Fig. 5a shows a mechanism of inhibiting activated natural killer cells and natural killer T cells or macrophages and a mechanism of inducing differentiation of immature dendritic cells to tolerogenic dendritic cells, all by means of tacrolimus.

As a result, it is possible to inhibit over-activated immunity before pregnancy or over-reactive immunity after pregnancy and restore a normal immune state, and in addition, blood type incompatible pregnancy or fetal hemochromatosis can be treated or ameliorated.

In detail, tacrolimus binds to FKBP52 in a chaperon complex of a progesterone receptor and then is activated by being released from the complex [Reference Literatures 33, 112, and 113].

Next, the activated progesterone receptor induces maturation of endometrial epithelial cells in the presence of progesterone. Galectin 1 is produced from these cells and induces Th1 apoptosis, tDC, and uterine NK in mature endometrial epithelial cells [Reference Literatures 30, 31, 34, and 35].

Secondly, tacrolimus directly inhibits the activity of the first type cells, NK and NKT cells, and macrophages via the CN-NFAT pathway [Reference Literatures 86 and 87].

Thirdly, tacrolimus induces differentiation of imDC and tDC via the CN-NFAT pathway and consequently assists in the induction of Treg cells [Reference Literatures 26, 51, 90, and 91].

Induction of tDC is also implemented by other pharmacological mediators, for example, immunosuppressive drugs (cyclosporine, rapamycin, deoxyspergurein, mofetil mycophenolate, and sanglifehrin A), anti-inflammatory drugs (corticosteroids and aspirin), 1α25-dihydroxyvitamin D3, N-acetyl-L-cysteine, cyclic AMP-inducing substances (PGE2, histamine, β2-agonists, and neuropeptides), glucosamine, and cobalt-protoporphyrin [Reference Literature 26].

Furthermore, the level of immunosuppression achieved by using tacrolimus for sterility and infertility is different from the use of tacrolimus for transplantation. The latter uses tacrolimus to achieve sustained immunosuppression at a level lower than ordinary immunity, and the former requires inhibiting over-activated immunity before pregnancy or over-reactive immunity after pregnancy and restoring a normal immune state (Fig. 5b).

### Blood type incompatible pregnancy

Blood type incompatible pregnancy is a disorder occurring in a state such as follows.
(i) An antigen that is absent on the red blood cell membrane of the mother's body exists on the red blood cell membrane of the fetus,
(ii) after the fetal blood flows into the mother's body via the placenta, a pathogen antibody to an antigen on the fetal red blood cell membrane is produced in the mother's body (an increase in the pathogen antibody, for example, the anti-D antibody titer, an anti-red blood cell antibody, and the like),
(iii) a pathogen antibody migrates to the fetus via the placenta and attacks fetal red blood cells, and
(iv) the fetal red blood cells hemolyze and cause fetal anemia.

Therefore, by administering the medicine of the present invention (for example, tacrolimus) to a pregnant woman from before the increase in the pathogen antibody titer as in (ii), for example, from immediately after conception, an increase in the pathogen body titer is suppressed (suppression of production of the antibody), symptoms caused by blood type incompatible pregnancy are suppressed, and therefore, it is possible to carry out treatment or amelioration of blood type incompatible pregnancy.

An example of the amount of administration is 1 mg/day to 10 mg/day in terms of the active ingredient. Preferably, the amount of administration is 3 to 6 mg/day.

Then, regarding the attack of fetal red blood cells by a pathogen antibody that has passed through the placenta as in (iii), since an antigen on the fetal red blood cell membrane is once (through pregnancy, miscarriage or the like of the previous time) recognized, and the memory for pathogen antibody production remains, blood type incompatible pregnancy is more likely to occur at the time of a pregnancy of the second and succeeding children than at the time of a pregnancy of the first child.

Furthermore, in a case where a woman suffers from sterility and infertility, and pregnancy has been established using, for example, the medicine of the present invention (1 to 4 mg/day in terms of the active ingredient), the medicine is continuously administered, and for example, when an increase in the pathogen body titer is recognized, the medicine may be administered after the amount of the medicine is further increased (for example, 5 to 10 mg/day in terms of the active ingredient).

Furthermore, even in a case where a woman suffers from sterility and infertility, and pregnancy has been established by, for example, a method other than the medicine of the present invention, treatment of blood type incompatible pregnancy using the medicine of the present invention is made possible, and for example, the medicine of the present invention (for example, 1 to 10 mg/day in terms of the active ingredient) may be administered from the early stage of pregnancy.

### Fetal hemochromatosis

Fetal hemochromatosis is a symptom occurring in a state such as follows.
(i) An enzyme related to iron metabolism of the fetus is different from that of the mother's body,
(ii) after fetal blood flows into the mother's body via the placenta, a pathogen antibody to the enzyme is produced in the mother's body,
(iii) the pathogen antibody migrates to the fetus via the placenta and attacks the iron metabolism enzyme of the fetus, and
(iv) iron metabolism of the fetus stops, and iron deposits in the liver to result in liver cirrhosis.

Therefore, by administering the medicine of the present invention (for example, tacrolimus) to a pregnant woman from before an increase in the pathogen antibody titer as in (ii), for example, from immediately after conception, an increase in the pathogen body titer is suppressed (suppression of production of antibody), symptoms caused by the pathogenic antibody are suppressed, and thereby, it is possible to carry out treatment or amelioration of fetal hemochromatosis.

The amount of administration of the compound as the active ingredient of the present invention at this time is preferably 1 to 10 mg/day, and more preferably 3 to 6 mg/day.

Pregnancy-induced hypertension syndrome and diseases associated therewith (HELLP syndrome, eclampsia, and the like)

In pregnancy-induced hypertension syndrome and diseases associated therewith (HELLP syndrome, eclampsia, and the like), both the mother's body and the fetus may be in a very dangerous state, such as fetal growth restriction, premature ablation of normally implanted placenta, fetal functional incompetence, and fetal death. That is caused by abnormal immunity between the mother's body and the fetus, and this abnormal immunity between the mother's body and the fetus is due to insufficient suppression of attack of cell-mediated immunity and humoral immunity by means of T cells on fetal components (antigens) including the placenta, or insufficient promotion of immunological tolerance.

Therefore, for example, by administering the medicine of the present invention (for example, tacrolimus) to a patient, suppression of activation of rejection immunity of the mother's body to the fetus and tolerance to the fetus can be promoted, more favorable placental construction and functions thereof are achieved, and it is possible to prevent and delay the onset of pregnancy-induced hypertension syndrome during pregnancy and diseases associated therewith.

Particularly, in a patient having a medical history of pregnancy-induced hypertension syndrome and diseases associated therewith, since recognition of fetal antigens after the first pregnancy is usually enhanced, it is preferable to administer the medicine of the present invention (for example, tacrolimus) to the patient even earlier upon the second and following pregnancies.

An example of the amount of administration is 1 mg/day to 10 mg/day in terms of the active ingredient. For example, since immunity of the mother's body to the fetus is strongly activated from the period where the inflow of fetal antigens to the mother's body increases, it is preferable to appropriately increase the amount of administration in the third trimester of pregnancy.

### Examples

Hereinafter, the present invention will be described by way of specific embodiments; however, the present invention is not intended to be limited to those embodiments, and it should be understood that various alterations and modifications in those embodiments can be carried out by those ordinarily skilled in the art, without deviating from the scope or purport of the present invention as defined in the attached claims.

### Measurement of anti-D antibody titer

The measurement is carried out by the indirect Coombs test. The indirect Coombs test is a test in which an anti-immunoglobulin antibody is added to a mixture of the blood serum of a patient and the blood of a healthy person to examine whether a hemagglutination reaction occurs (any irregular antibody present in the blood serum is detected) (for the Coombs test, see Nissan Women's Journal Vol. 59, No. 10, N-617-N-623).

### Th1/Th2 cell ratio

In recent years, the incidence of in vitro fertilization (IVF) and embryo transplantation (ET) is increasing all over the world. Along with this, the number of women experiencing multiple IVF failures, including repeated implantation failure, is increasing. Upon conducting IVF/ET, embryos are transplanted into the uterine cavity within 2 to 5 days after fertilization. Pregnancy is established when a so-called semi-allograft embryo is successfully implanted on the maternal decidua concomitantly with the establishment of immunological tolerance on the mother's body side [Reference Literature 7a]. Establishing an appropriate immune response at the time of implantation is the key to successful implantation. Therefore, immunological etiology is considered to play an important role in RIF after IVF/ET.

T helper (Th)1, Th2, Th17, and Treg cells accomplish important roles in regard to immune responses such as, for example, immunological rejection and immunological tolerance [Reference Literature 8a]. It is generally agreed that the immune state during pregnancy is associated with Th2 dominance and the Th1 immune response is associated with embryonic rejection [Reference Literatures 6a and 9a]. The underlying mechanism of embryonic rejection is considered to be similar to a rejection reaction in allograft [Reference Literature 10a]. Embryos transplanted during IVF/ET may fail to implant due to an immune response similar to a rejection reaction in allograft.

### Analysis of Th1 cells and Th2 cells

For the purpose of evaluating the baseline value of the Th1/Th2 cell ratio, a total of 10 ml of venous blood was collected. Th1 cells and Th2 cells were determined by detecting the production of intracellular interferon (IFN)-γ and IL-4.

Specific staining of lymphocytes was performed by incubating whole blood together with anti-CD4-PC5 or anti-CD8-PC5-conjugated monoclonal antibody (mAbs) (Beckman Coulter, Inc., Fullerton, Ca, USA). Red blood cells (RBCs) were removed by hemolysis (using FACS Lysing solution; Becton, Dickinson and Company, BD 134 Biosciences, Franklin Lake, NJ, USA), and lymphocytes were analyzed using flow cytometry (FACSCalibur; Becton, Dickinson and Company). Activated whole blood samples were surface-stained using anti-CD4-PC5-conjugate mAbs, and then RBC hemolysis and specific intracellular staining using Fast Immune (trademark) IFN-γ-FITC/IL-4-PE (Becton, Dickinson and Company) were carried out in sequence according to the manufacturer's instructions for use. Th1 cells were defined as CD4⁺ lymphocytes accompanied by intracellular IFN-γ but not by intracellular IL-4. Th2 cells were detected as CD4⁺ lymphocytes accompanied by intracellular IL-4 but not by intracellular IFN-γ. The ratio of intracellular IFN-γ-positive Th cells with respect to intracellular IL-4-positive Th cells was expressed as the Th1/Th2 cell ratio.

### Example 1

Treatment of blood type incompatible pregnancy using tacrolimus by inhibiting over-activated immunity before pregnancy or over-reactive immunity after pregnancy and restoring normal immune state

The patient is a 35-year-old woman having blood type A. The patient suffering from a blood type incompatible pregnancy with negative maternal Rho (D) and positive fetal Rho (D) was subjected to the following treatments. That is, at the time of the first pregnancy, anti-D immunoglobulin was not administered during pregnancy, sensitization was effected before delivery, and the anti-D antibody titer at the time of delivery was 8-fold. In week 39, she gave birth by emergency cesarean section due to placental abruption of the upper placenta. After delivery, the antibody titer obtained after 5 months showed a maximum value of 64-fold.

Two years later, she hoped to have a second child but suffered from infecundity and was treated for sterility by in vitro fertilization; however, since the treatment was unsuccessful after five embryo transplantations, a close examination of the immune system was conducted. At that time, a marked increase (24.9) in the Th1/Th2 (Th1 32.4, Th2 1.3) ratio was recognized, the illness was judged as infecundity considered to be caused by an abnormality in the immune system, and a tacrolimus treatment was selected. Pregnancy was established by a single embryo transplantation with a treatment using 4 mg/day of tacrolimus (oral administration: 2 mg in the morning, 2 mg in the evening).

The anti-D antibody titer obtained immediately after the establishment of pregnancy was 4-fold; however, the titer reached 16-fold in week 24 and reached 32-fold in week 26 (Fig. 6, upper part). After that, migration of fetal antigens to the mother's body via the placenta was assumed to further increase, and preparation of plasmapheresis and fetal blood transfusion was initiated in preparation for a rapid increase in the antibody titer. Furthermore, at the same time, the dose of tacrolimus was increased to 5 mg/day (oral administration: 3 mg in the morning and 2 mg in the evening), in consideration of the fact that Th1 in week 28 showed a tendency of increase again.

Since then, the expected increase in the anti-D antibody titer did not occur at all, the 32-fold was maintained without observing any fetal anemia (increased blood flow velocity in the middle cerebral artery), growth of the child occurred without any problem (Fig. 7), and the patient gave birth to a healthy boy who weighed 2834 g on day 2 of week 37. Furthermore, the blood flow velocity in the middle cerebral artery and the estimated body weight of the fetus were measured by fetal ultrasonography.

Fig. 7 is a graph showing the change in the body weight of a fetus during the weeks of pregnancy. The body weight is increasing over time, which shows that the fetus is growing correspondingly to the number of weeks of pregnancy. At the same time, Fig. 7 also shows the blood flow velocity in the middle cerebral artery and shows that fetal anemia was not developed during the entire course of pregnancy.

The concentration of tacrolimus in the cord blood was lower than or equal to the detection limit value according to chemiluminescence immunoassay (ECLIA), and the anti-D antibody titer was double. The blood type of the child was type A Rho (D) positive, the Hb (hemoglobin) level at the time of birth was 13.6, which was a slightly low value, and there were no external malformations or visceral malformations and no problem with physical functions.

### Analysis

Fetal hemoglobin can exist in maternal blood from the early stage of pregnancy. HbF in maternal blood is detected in the early stages of pregnancy, and the migration of HbF in fetal blood to maternal blood is generally observed from about 9 weeks of pregnancy [Reference Literatures 4a, 11a, and 12a]. These findings suggest that the maternal immune response to fetal antigens can occur from the early stage of pregnancy, in which inflow of the fetal antigens into the mother's body begins. On the other hand, the migration of anti-D antibody to the fetus begins after completion of the placental construction.

Since this patient did not have anti-D immunoglobulin having neutralizing activity, administered to the mother's body during the first pregnancy, there was a possibility that a large amount of fetal blood flowed into the mother's body during delivery due to placental abruption of the upper placenta, and it is considered that sensitization to the fetal antigens was strongly established. In addition, there is a high possibility that a general sterility treatment did not result in pregnancy and recurrent failures further promoted sensitization. A possibility was considered in which due to these happenings, fetal antigens including blood cell components were strongly recognized by the mother's body, and rejection caused by not only humoral immunity represented by anti-D antibody but also cell-mediated immune response to other fetal components due to a marked increase in the Th1 cell ratio, began from the second pregnancy activity and caused infecundity.

The elevated anti-D antibody titer and the increased Th1 cell ratio were induced by humoral immunity and cell-mediated immunity, respectively These are the conventional immune responses to exogenous antigens and fetal antigens.

The inventors could treat patients using tacrolimus in order to inhibit over-activated immunity before pregnancy or over-reactive immunity after pregnancy and restore a normal immune state, and suppress cell-mediated immunity against infecundity. Detailed mechanisms and results related to Th1 cells have been previously described [Reference Literatures 13a to 15a]. Based on these results, administration of tacrolimus was initiated before pregnancy as a treatment for infecundity, administration was continued because a decreasing tendency was not observed in the Th1 cell ratio even after the establishment of pregnancy, and the dose was increased to 5 mg/day because the Th1 cell ratio increased in week 28 of pregnancy. The maternal and fetal conditions were stable without complications, the pregnancy progressed favorably, and the patient achieved a safe delivery.

After week 24 of pregnancy, there was a possibility that the anti-D antibody titer could rise sharply in response to the invasion of a large amount of fetal red blood cells; however, consequently, the production of anti-D antibody was also suppressed, and intensive care for the mother's body and the child was unnecessary until delivery.

Although this effect was unexpected, the mechanism can be described such that recognition of antigens and production of anti-pathogen antibodies are inhibited by down-regulation of the T cell function by inhibiting the calcineurin-NFAT pathway, and subsequent inhibition of B cell activation and inhibition of antibody production. This can also be thought of as continuous treatment using high doses of intravenous immunoglobulin having an immunosuppressive effect [Reference Literatures 16a to 19a].

In conclusion, this treatment provides a benefit of alleviating the promotion of antibody production without conducting intensive treatments such as plasmapheresis, large-quantity γ-globulin therapy, and high-dose steroid therapy. The inventors believe that treatment using tacrolimus is beneficial for allogeneic immune pregnancies.

It is thought that pregnancy was easily established by immunosuppression by administration of tacrolimus, production of anti-D antibody was suppressed, together with stable continuation of pregnancy, by increasing the amount of tacrolimus during pregnancy for further enhancement of rejection immunity, and it was possible to avoid the onset of fetal anemia. It was speculated that this was due to an effect brought by suppression of both cell-mediated immunity and humoral immunity by tacrolimus.

### Example 2

Forty-two patients who had a medical history of five consecutive incidences of RIF after in vitro fertilization and embryo transplantation using morphologically and developmentally good-quality embryos, and for whom abnormal findings related to sterility and infertility were not recognized in serological tests, were treated from two days before the embryo transplantation to the day of pregnancy test, using or without using tacrolimus. The daily dose of tacrolimus was determined according to the Th1/Th2 cell ratio. The clinical pregnancy rate of the patients who had received treatment was 64.0% (16/25) as compared to 0% (0/17) of an untreated group [Reference Literature 114]. It is understood that the Th1/Th2 cell ratio before embryo transplantation can predict the results of assisted reproductive technology in tacrolimus-treated and tacrolimus-untreated patients, and the Th1 cell level is negatively correlated with the pregnancy results (n = 124) [Reference Literature 115].

With regard to RPL, a patient of a case having a history of eleven consecutive miscarriages in weeks 5 to 8 of pregnancies was treated with low-dose aspirin, heparin, prednisolone (5 mg/day), and large-quantity γ-globulin therapy (1 g/kg for 3 days) in this order; however, the treatment was not successful [Reference Literature 116]. However, by continuously treating the patient with tacrolimus (2 mg/day) during pregnancy, the patient followed favorable pregnancy progress without having other treatments. In addition, with regard to immunosuppression of B cells using tacrolimus, satisfactory pregnancy progress was obtained without using plasma exchange for RhD incompatible pregnancy, and the patient succeeded in giving birth (data not shown). The mechanism of tacrolimus mainly depends on the suppression of cell-mediated immunity but also suppresses humoral immunity by means of T cells.

Tacrolimus can affect other diseases caused by abnormality of immunity between the mother's body and the fetus as well as construction and functions of placenta, and tacrolimus can prevent retarded fetal growth during pregnancy and pregnancy-induced hypertension in the mother's body.

From the viewpoint of suppressing humoral immunity, tacrolimus can be a candidate drug for the treatment of neonatal hemochromatosis (this is attributable to humoral immunity reactions to fetal antigens such as RhD incompatible pregnancy) and antiphospholipid syndrome, without adopting strong treatments such as plasma exchange therapy and large-quantity gamma-globulin therapy during pregnancy.

However, in the case of a pathological condition caused by existing antibodies after pregnancy, since tacrolimus only suppresses differentiation of B cells, in order to rapidly remove the activity of existing pathogenic antibodies, combined therapy with these strong treatments is required.

Tacrolimus treatment is particularly beneficial for use in patients having a medical history of recurrent miscarriage or recurrent chemical abortion in the early stage of pregnancy, that is, before from weeks 5 to 6 of pregnancy.

It is difficult to determine the optimum timing on the occasion of performing strong treatment such as glucocorticoid in an amount at an immunosuppressive level or large-quantity γ-globulin therapy. Tacrolimus will be easily used even at such times.

Furthermore, while Th1-dominant immunity is explained, it is difficult to describe the mechanism of rejection immunity in sterility and infertility patients having high Th2 cell ratios; however, when it is assumed that rejection reactions depend on antibodies to fetal HLA, tacrolimus can be effective in these cases.

### Example 3

### Treatment of neonatal hemochromatosis

The main purpose of the present therapeutic method was to suppress the recognition of fetal antigens in the mother's body and to lower the ability to produce pathogen antibodies.

Although the diseases developed in the fetus are different, as in the case of blood type incompatible pregnancy, fetal antigens flow in from the fetus into the mother's body after the completion of placental construction and are recognized in the mother's body, pathogen antibodies against those antigens are produced, and IgG among them migrates to the fetus via the placenta and thereby causes diseases in the fetus. Regarding the present disease, these pathogen antibodies inhibit proteins related to fetal iron metabolism, iron is deposited in the liver, resulting in liver failure, and in many cases, the disease may be severe to the extent that leads to intrauterine fetal death or postnatal death or requires liver transplantation.

The mechanism of this treatment can be described such that recognition of fetal antigens and production of anti-pathogen antibodies are inhibited by suppression of T cell functions caused by inhibition of the calcineurin/NFAT pathway, as well as consequent inhibition of B cell activation and suppression of antibody production.

A fetal antigen that is considered responsible for this disease has not been identified. Assuming that a causative antigen flows in from the fetus into the mother's body after completion of the placenta construction, initiation of treatment from around week 12 of pregnancy may be considered; however, since there are individual differences in the placental construction, and the timing for the migration of fetal antigens is not clear, it is preferable to initiate treatment from the early stage of pregnancy in order to conduct more effective treatment.

In the case of large-quantity γ-globulin therapy for the mother's body, which is the only existing preventive method, it is necessary to initiate the therapy from week 18 and continue administration until delivery, and the medical cost (600,000 yen/week) is extremely high; however, the present treatment method allows treatment to be performed during the entire course of pregnancy at a medical cost of 1/10 or less of the aforementioned cost, while it is also easy to change the amount of administration as appropriate, so that there is a possibility that the medical cost can be further saved.

In the large-quantity γ-globulin therapy for the mother's body, which is the only existing preventative method, since purification is performed from a large amount of collected and pooled blood, there is a risk of being exposed to infections that is highly possibly included in the blood, and particularly parvovirus infection that causes fetal anemia is considered as a problem; however, there is no risk of infection including other viruses in the present treatment method.

In fetal hemochromatosis, during pregnancy, any of miscarriage, premature birth, intrauterine growth restriction, oligohydramnios, fetal movement insufficiency, or placental edema is frequently recognized, and after birth, defective systemic condition from immediately after birth (respiratory and circulatory failure, and the like), retarded fetal growth, hydrops fetalis, signs of liver failure, and the like are recognized. In neonatal hematological findings, coagulopathy, cholestasis, abnormal transaminase levels, and the like are observed. Disseminated intravascular coagulation syndrome not caused by sepsis, high ferritin level high α-fetoprotein level, high transferrin saturation rate are shown, and in image inspection findings, low signals suggesting iron deposition in organs other than the liver are recognized by MRI T2-weighted imaging. Through this treatment, evaluation is enabled by improving these findings for the fetus during pregnancy and the infant after birth.

It is difficult to determine the intrauterine immunological condition based on the information of peripheral blood of the mother's body. In several studies, the correlation between peripheral blood and the NK cell level in the decidua has been evaluated, and there are also conflicting opinions [Reference Literatures 117 and 118]. When it is assumed that the immune state of the uterus of an RIF patient reflects a normal immune state of the whole body, it may be simple to judge the uterine immune state of an RIF patient as compared to an RPL patient. In contrast, it may take time until the maternal peripheral blood of an RPL Patient to reflect the uterine state, and in a patient with early RPL, there is a possibility that the immune state of maternal peripheral blood may undergo no change or very weak changes. Depending on cases, changes may occur after abortion. A problem with tacrolimus treatment in these patients is that there is no change in the cell ratio in the peripheral blood after tacrolimus treatment, and there is even a possibility that the activity may be suppressed in the uterus.

Several immunological parameters have been evaluated in the maternal blood. However, those do not directly reflect the fetal state, and it is implied that only the immunological state of the mother's body and its reactivity to fetal antigens can be analyzed. In order to support an accurate and appropriate method of using an immunosuppressant, and to provide more detailed information on the fetus, it is necessary to carry out further research.

Unlike the pathological conditions of infecundity and infertility, the ordinary maternal immune state of infecundity is reflected in the uterus; however, in infertility, it is speculated that the intrauterine immune reactions are not rapidly transmitted to the whole body, and in many cases, pregnancy is discontinued before the intrauterine immune reactions exert any change in the whole body. Therefore, a new biomarker is needed.

### Example 4

Prevention of onset or delay of onset of pregnancy-induced hypertension syndrome and diseases associated therewith (HELLP syndrome and eclampsia)

A main purpose of the present treatment method is to promote suppression of activation of rejection immunity of the mother's body to the fetus and tolerance to the fetus.

A patient is a woman at age 38 having a medical history of pregnancy-induced hypertensive nephropathy and HELLP syndrome. At the time of the first pregnancy at age 32, the patient complained of a poor physical condition from the early stage of pregnancy and showed tendencies of pedal edema, weight gain, and elevation of blood pressure from week 15 of pregnancy, rapid enhancement of edema, proteinuria, and hypertension appeared from week 18 of pregnancy, and thus the patient was diagnosed with preeclampsia (PE) from among the pregnancy-induced hypertension syndrome (HDP). The patient developed HELLP syndrome and eclamptic attack in week 20 of pregnancy, was subjected to an urgent Caesarian section, and had a stillborn baby. After the delivery, pulmonary edema, disseminated intravascular coagulation (DIC), and intraperitoneal bleeding, all of which are severe complications, were recognized, and the patient received treatments such as management using an artificial respirator, supplement of albumin, and blood component transfusion.

This time, in the hematologic examination findings before pregnancy, there were no abnormalities in all of the liver function, kidney function, and coagulation function, and abnormal findings were not observed in the examination items involved in infertility such as antiphospholipid antibody syndrome (anti-CL-IgG antibody, anti-CL-IgM antibody, anti-PS/PT antibody, anti-CL-β2GP1 antibody, LAC, anti-PE-IgG antibody, and anti-PE-IgM antibody), autoimmune diseases (anti-DNA antibody and antinuclear antibody), and coagulation dysfunction.

The circumstances of the first pregnancy and the pregnancy of this time are described in Table 1.

**(Table 1)**

| | | First pregnancy (without tacolimus treatment) | | Pregnancy of this time (with tacrolimus treatment) | |
|---|---|---|---|---|---|
| | | Before parturition | After parturition | Before parturition | After parturition |
| Symptoms | Edema | Conspicuous | Conspicuous | Negligible | Negligible |
| | Proteinuria initiated (weeks of pregnancy) | 18 | | 33 | |
| | Hypertension | Conspicuous | Conspicuous | Not observed | Temporary |
| | Complications after parturition | | - Pulmonary edema | | None |
| | | | - DIC | | |
| | | | - Associated with infection Intraperitoneal bleeding | | |
| Treatment | | - Calcium antagonist drug | - Calcium antagonist drug | None | - Magnesium sulfate (perioperative) |
| | | | - Magnesium sulfate | | |
| | | - Magnesium sulfate | - Management using artificial respirator | | |
| | | | - Supplement of albumin | | |
| | | | - Component transfusion (RBC, FFP, PLT) | | |
| Blood examination | AST (U/L) | 1141 | 959 | 65 | 898 |
| | ALT (U/L) | 891 | 729 | 71 | 833 |
| | LDH (U/L) | 1968 | 1857 | 316 | 1995 |
| | Cr (mg/dL) | 1 | 0.96 | 1 | 0.59 |
| | WBC (mL) | 12200 | 11700 | 8280 | 8220 |
| | Hb (g/dL) | 14.9 | 12.6 | 11 | 10.5 |
| | Plt (10⁴/mL) | 4.2 | 2.8 | 10 | 2.2 |
| Information on fetus | Gestation period | | Week 20 day 0 | | Week 33 day 3 |
| | Body weight (g) | | 138 | | 1490 |
| | Fetal growth | | - 2.0 SD | | - 1.9 SD |

| | | | | | |
|---|---|---|---|---|---|
| AST: Aspartate aminotransferase ALT: Alanine aminotransferase LDH: Lactate dehydrogenase Cr: Creatinine WBC: Number of white blood cells Hb: Hemoglobin Plt: Number of blood platelets | | | | | |

The ratio of Th1 (CD4g⁺IFN-g⁺)/Th2 (CD4⁺IL-4⁺) in CD4-positive cells is 15.2/2.1 (normal value: less than 10.3), and clear immunological abnormalities were also not observed before pregnancy [Reference Literature 12b]; however, it was assumed that after implantation of a fertilized ovum, immunity of the mother's body would be activated, and immune abnormality between the mother's body and the fetus would suppress placental construction and functions thereof, causing fetal growth restriction and hypertension during pregnancy. Considering this, the patient was treated with 1 mg/day of tacrolimus from week 4 of pregnancy, for the purpose of controlling the immunity between the mother's body and the fetus after confirmation of pregnancy.

The pregnancy progress was smooth, proteinuria, edema, and hypertension were not observed until week 32 of pregnancy, and growth of the fetus was also favorable; however, the patient showed a tendency of proteinuria and elevation of blood pressure in the next week, rapidly acquired HELLP syndrome for about subsequent 3 days, and gave birth to a boy weighing 1490 g by a Caesarean section on day 3 of week 33 of pregnancy.

In the immunological findings during the pregnancy progress, the ratio of Th1 and Th2 cells among CD4-positive cells continuously decreased, while the NK cell activity (normal value: 18% to 40%) decreased in the second trimester and then conspicuously increased from week 32 of pregnancy (Fig. 8).

In this case, monotherapy was carried out with 1 mg/day of tacrolimus from the early stage of pregnancy, and combined treatment of low dose aspirin (LDA) intended for HDP prevention by bloodstream improvement was not carried out [Reference Literature 13b]. Regarding the pregnancy progress, there was no problem in the second trimester of pregnancy, where the patient developed HELLP syndrome in the previous pregnancy, and pregnancy progressed smoothly to the third trimester of pregnancy; however, the patient rapidly acquired HELLP syndrome similarly to the previous pregnancy, after week 33 of pregnancy.

In the immunological examination findings, both the ratio of Th1 and Th2 cells among CD4-positive cells and the NK cell activity decreased until the second trimester, and sufficient immunosuppression by tacrolimus was observed; however, in week 32 of pregnancy, the NK cell activity was increasing.

As one possibility, it was suggested that the immunity of the mother's body to the fetus was strongly activated from the timing when the inflow of fetal antigens to the mother's body increased, and the immunosuppressive action by 1 mg/day of tacrolimus became insufficient in the third trimester of pregnancy. Furthermore, a possibility in which T cells other than Treg were simultaneously activated in a state in which the cell proportions did not change, was also considered.

Two times of HELLP syndrome developed in the same patient; however, it is easily speculated that the onset mechanism was similar. Examples of severe complications of HDP include HELLP syndrome and eclampsia; however, this patient was untreated and developed both diseases in early pregnancy during the previous pregnancy and had a stillborn baby. This time the patient acquired HELLP syndrome; however, early onset was avoided by single administration of tacrolimus, the onset was delayed for about 13 weeks compared to the previous pregnancy, and the patient succeeded in childbearing.

In sterility and infertility patients involving immune abnormality, usually there is a possibility that recognition of fetal antigens after the first pregnancy may be enhanced. Therefore, it was speculated that HELLP syndrome and eclamptic attack associated with aggravation of pregnancy-induced hypertension in the subsequent pregnancy would be developed earlier. On the contrary, it was successful to delay the onset time compared to the previous pregnancy through the treatment with tacrolimus, and the effect became clear by the absence of a concomitant drug. It was conceived that by promoting suppression of activation of rejection immunity of the mother's body to the fetus and tolerance to the fetus, more favorable placental construction and functions thereof were achieved, and prevention of hypertension during pregnancy and fetal growth restriction were achieved. However, when the factor that pregnancy could not be continued to the maturity and the perinatal growth of the infant was also late was taken into consideration, it was conceived that a larger amount of administration was needed in this case.

In this case, we discussed the pathological condition of patients with the proportions of Th1 and Th2 cells among CD4-positive cells and the NK cell activity and suggested expression of abnormal immunity between the mother's body and the fetus using one of them, that is, the NK cell activity; however, there are available more parameters such as Treg cells, Tfh cells, and Th17 cells, which adopt abnormality of immunological tolerance of the mother's body to fetal antigens, enhancement of rejection immunity, and subsequent inflammation as biomarkers, as well as cytokines and chemokines, for the evaluation of pregnancy-induced hypertension [Reference Literatures 3b to 10b].

Evaluation by means of more parameters is useful for understanding the state of the mother's body in detail, and it is expected to attain discovery of new parameters for evaluating the immune state between the mother's body and the fetus and an increase in subjects for evaluating a therapeutic effect for pregnancy-induced hypertension syndrome and diseases associated therewith.

### Industrial Applicability

Regarding the variation in the maternal immunity before and after a pregnancy, (1) a state in which the maternal immunity is activated at a higher level compared to before pregnancy, (2) a state in which the maternal immunity is activated by recognizing fetal antigens for the first time in the early stage of pregnancy, and (3) a state in which the amount of fetal antigens increases after the second trimester of pregnancy, and the maternal immunity is activated, are suspended. According to the present invention, it is possible to inhibit over-activated immunity before pregnancy or over-reactive immunity after pregnancy and to restore a normal immune state, and as a result, autoimmune diseases including not only sterility and infertility in which cell-mediated immunity (natural immunity and acquired immunity) in a materno-fetal relationship is involved, but also sterility and infertility in which humoral immunity is involved, and antiphospholipid antibody syndrome; blood type incompatible pregnancy, fetal hemochromatosis, or the like can be treated or ameliorated, so that continuation of pregnancy and childbirth of a healthy baby are made possible. Furthermore, as these immune states between the mother's body and the fetus are favorably maintained, it is possible to achieve placental construction favorably, and this also leads to avoidance of maternal complications (pregnancy-induced hypertension syndrome and diseases associated therewith, namely, HELLP syndrome and eclampsia).

### (Citation List)

1: Inhorn MC, Patrizio P. Infertility around the globe: new thinking on gender, reproductive technologies and global movements in the 21st century. Hum Reprod Update. 2015;21:411-26.
2: Vannuccini S, Clifton VL, Fraser IS, Taylor HS, Critchley H, Giudice LC, Petraglia F. Infertility and reproductive disorders: impact of hormonal and inflammatory mechanisms on pregnancy outcome. Hum Reprod Update. 2016;22:104-15.
3: Abdolmohammadi-Vahid S, Danaii S, Hamdi K, Jadidi-Niaragh F, Ahmadi M, Yousefi M. Novel immunotherapeutic approaches for treatment of infertility. Biomed Pharmacother. 2016;84:1449-1459.
4: Colf LA, Bankovich AJ, Hanick NA, Bowerman NA, Jones LL, Kranz DM, Garcia KC. How a single T cell receptor recognizes both self and foreign MHC. Cell. 2007 Apr 6;129:135-46.
5: Felix NJ, Donermeyer DL, Horvath S, Walters JJ, Gross ML, Suri A, Allen PM. Alloreactive T cells respond specifically to multiple distinct peptide-MHC complexes. Nat Immunol. 2007;8:388-97.
6: Borbulevych OY, Piepenbrink KH, Gloor BE, Scott DR, Sommese RF, Cole DK, Sewell AK, Baker BM. T cell receptor cross-reactivity directed by antigen-dependent tuning of peptide-MHC molecular flexibility. Immunity. 2009;31:885-96.
7: Macdonald WA, Chen Z, Gras S, Archbold JK, Tynan FE, Clements CS, Bharadwaj M, Kjer-Nielsen L, Saunders PM, Wilce MC, Crawford F, Stadinsky B, Jackson D, Brooks AG, Purcell AW, Kappler JW, Burrows SR, Rossjohn J, McCluskey J. T cell allorecognition via molecular mimicry. Immunity. 2009;31:897-908.
8: Conlon TM, Saeb-Parsy K, Cole JL, Motallebzadeh R, Qureshi MS, Rehakova S, Negus MC, Callaghan CJ, Bolton EM, Bradley JA, Pettigrew GJ. Germinal center alloantibody responses are mediated exclusively by indirect-pathway CD4 T follicular helper cells. J Immunol. 2012;188:2643-52.
9: Martins PN, Tullius SG, Markmann JF. Immunosenescence and immune response in organ transplantation. Int Rev Immunol. 2014;33:162-73.
10: Li H, Shi B. Tolerogenic dendritic cells and their applications in transplantation. Cell Mol Immunol. 2015;12:24-30.
11: Moreau A, Alliot-Licht B, Cuturi MC, Blanco G. Tolerogenic dendritic cell therapy in organ transplantation. Transpl Int. 2017;30:754-764.
12: Marin E, Cuturi MC, Moreau A. Tolerogenic Dendritic Cells in Solid Organ Transplantation: Where Do We Stand? Front Immunol. 2018;9:274.
13: Gallois A, Bhardwaj N. Dendritic cell-targeted approaches to modulate immune dysfunction in the tumor microenvironment. Front Immunol. 2013 Dec 10;4:436. doi: 10.3389/fimmu.2013.00436.
14: Borst J, Ahrends T, Cabala N, Belief CJM, Kastenmueller W. CD4+ T cell help in cancer immunology and immunotherapy. Nat Rev Immunol. 2018. doi: 10.1038/s41577-018-0044-0. [Epub ahead of print]
15: Shitara K, Nishikawa H. Regulatory T cells: a potential target in cancer immunotherapy. Ann N Y Acad Sci. 2018;1417:104-115.
16: Ahrends T, Borst J. The opposing roles of CD4+ T cells in anti-tumour immunity. Immunology. 2018. doi: 10.1111/imm.12941. [Epub ahead of print]
17: Freeman GJ, Long AJ, Iwai Y, Bourque K, Chernova T, Nishimura H, Fitz LJ, Malenkovich N, Okazaki T, Byrne MC, Horton HF, Fouser L, Carter L, Ling V, Bowman MR, Carreno BM, Collins M, Wood CR, Honjo T. Engagement of the PD-1 immunoinhibitory receptor by a novel B7 family member leads to negative regulation of lymphocyte activation. J Exp Med. 2000;192:1027-34.
18: Latchman Y, Wood CR, Chernova T, Chaudhary D, Borde M, Chernova I, Iwai Y, Long AJ, Brown JA, Nunes R, Greenfield EA, Bourque K, Boussiotis VA, Carter LL, Carreno BM, Malenkovich N, Nishimura H, Okazaki T, Honjo T, Sharpe AH, Freeman GJ. PD-L2 is a second ligand for PD-1 and inhibits T cell activation. Nat Immunol. 2001;2:261-8.
19: Kovats S, Main EK, Librach C, Stubblebine M, Fisher SJ, DeMars R. A class I antigen, HLA-G, expressed in human trophoblasts. Science. 1990;248:220-3.
20: Hiby SE, King A, Sharkey A, Loke YW. Molecular studies of trophoblast HLA-G: polymorphism, isoforms, imprinting and expression in preimplantation embryo. Tissue Antigens. 1999;53:1-13.
21: Goodridge JP, Witt CS, Christiansen FT, Warren HS.KIR2DL4 (CD158d) genotype influences expression and function in NK cells. J Immunol. 2003;171:1768-74.
22: LeMaoult J, Zafaranloo K, Le Danff C, Carosella ED. HLA-G up-regulates ILT2, ILT3, ILT4, and KIR2DL4 in antigen presenting cells, NK cells, and T cells. FASEB J. 2005;19:662-4.
23: Hviid TV. HLA-G in human reproduction: aspects of genetics, function and pregnancy complications. Hum Reprod Update. 2006;12:209-32.
24: Shakhawat A, Shaikly V, Elzatma E, Mavrakos E, Jabeen A, Fernandez N. Interaction between HLA-G and monocyte/macrophages in human pregnancy. J Reprod Immunol. 2010;85:40-6.
25: Rajagopalan S. HLA-G-mediated NK cell senescence promotes vascular remodeling: implications for reproduction. Cell Mol Immunol. 2014;11:460-6.
26: Morelli AE, Thomson AW. Tolerogenic dendritic cells and the quest for transplant tolerance. Nat Rev Immunol. 2007;7:610-21.
27: Lydon JP, DeMayo FJ, Funk CR, Mani SK, Hughes AR, Montgomery CA Jr, Shyamala G, Conneely OM, O'Malley BW. Mice lacking progesterone receptor exhibit pleiotropic reproductive abnormalities. Genes Dev. 1995; 9: 2266-78.
28: Todd A. Tibbetts, Franco DeMayo, Susan Rich, Orla M. Conneely, Bert W. O'Malley. Progesterone receptors in the thymus are required for thymic involution during pregnancy and for normal fertility. Proc Natl Acad Sci U S A. 1999; 96: 12021-12026.
29: Hirota Y, Cha J, Dey SK. Revisiting reproduction: Prematurity and the puzzle of progesterone resistance. Nat Med. 2010;16:529-31.
30: Terness P, Kallikourdis M, Betz AG, Rabinovich GA, Saito S, Clark DA. Tolerance signaling molecules and pregnancy: IDO, galectins, and the renaissance of regulatory T cells. Am J Reprod Immunol. 2007;58:238-54.
31: Than NG, Romero R, Erez O, Weckle A, Tarca AL, Hotra J, Abbas A, Han YM, Kim SS, Kusanovic JP, Gotsch F, Hou Z, Santolaya-Forgas J, Benirschke K, Papp Z, Grossman LI, Goodman M, Wildman DE. Emergence of hormonal and redox regulation of galectin-1 in placental mammals: implication in maternal-fetal immune tolerance. Proc Natl Acad Sci U S A. 2008;105:15819-24.
32: Molvarec A, Blois SM, Stenczer B, Toldi G, Tirado-Gonzalez I, Ito M, Shima T, Yoneda S, Vasarhelyi B, Rigo J Jr, Saito S. Peripheral blood galectin-1-expressing T and natural killer cells in normal pregnancy and preeclampsia. Clin Immunol. 2011;139:48-56.
33: Hirota Y, Burnum KE, Acar N, Rabinovich GA, Daikoku T, Dey SK. Galectin-1 markedly reduces the incidence of resorptions in mice missing immunophilin FKBP52. Endocrinology. 2012;153:2486-93.
34: Ramhorst RE, Giribaldi L, Fraccaroli L, Toscano MA, Stupirski JC, Romero MD, Durand ES, Rubinstein N, Blaschitz A, Sedlmayr P, Genti-Raimondi S, Fainboim L, Rabinovich GA. Galectin-1 confers immune privilege to human trophoblast: implications in recurrent fetal loss. Glycobiology. 2012;22:1374-86.
35: Barrientos G, Freitag N, Tirado-Gonzalez I, Unverdorben L, Jeschke U, Thijssen VL, Blois SM. Involvement of galectin-1 in reproduction: past, present and future. Hum Reprod Update. 2014;2:175-93.
36: Than NG, Romero R, Balogh A, Karpati E, Mastrolia SA, Staretz-Chacham O, Hahn S, Erez O, Papp Z, Kim CJ. Galectins: Double-edged Swords in the Cross-roads of Pregnancy Complications and Female Reproductive Tract Inflammation and Neoplasia. J Pathol Transl Med. 2015;49:181-208.
37: El Costa H, Tabiasco J, Berrebi A, Parant O, Aguerre-Girr M, Piccinni MP, Le Bouteiller P. Effector functions of human decidual NK cells in healthy early pregnancy are dependent on the specific engagement of natural cytotoxicity receptors. J Reprod Immunol. 2009;82:142-7.
38: Karami N, Boroujerdnia MG, Nikbakht R, Khodadadi A. Enhancement of peripheral blood CD56(dim) cell and NK cell cytotoxicity in women with recurrent spontaneous abortion or in vitro fertilization failure. J Reprod Immunol. 2012;95:87-92.
39: Aluvihare VR, Kallikourdis M, Betz AG. Regulatory T cells mediate maternal tolerance to the fetus. Nat Immunol. 2004;5:266-71.
40: Guerin LR, Prins JR, Robertson SA. Regulatory T-cells and immune tolerance in pregnancy: a new target for infertility treatment? Hum Reprod Update. 2009;15:517-35.
41: Shima T, Sasaki Y, Itoh M, Nakashima A, Ishii N, Sugamura K, Saito S. Regulatory T cells are necessary for implantation and maintenance of early pregnancy but not late pregnancy in allogeneic mice. J Reprod Immunol. 2010;85:121-9.
42: Saito S, Nakashima A, Shima T, Ito M. Th1/Th2/Th17 and regulatory T-cell paradigm in pregnancy. Am J Reprod Immunol. 2010;63:601-10.
43: Saito S, Shima T, Nakashima A, Inada K, Yoshino O. Role of Paternal Antigen-Specific Treg Cells in Successful Implantation. Am J Reprod Immunol. 2016;75:310-6.
44: Loke, Y.W., and A. King. 1995. Human Implantation: Cell Biology and Immunology. Cambridge University Press, Cambridge,UK. 299 pp.
45: King A, Burrows TD, Hiby SE, Bowen JM, Joseph S, Verma S, Lim PB, Gardner L, Le Bouteiller P, Ziegler A, Uchanska-Ziegler B, Loke YW. Surface expression of HLA-C antigen by human extravillous trophoblast. Placenta. 2000;21:376-87.
46: Hiby SE, Walker JJ, O'shaughnessy KM, Redman CW, Carrington M, Trowsdale J, Moffett A. Combinations of maternal KIR and fetal HLA-C genes influence the risk of preeclampsia and reproductive success. J Exp Med. 2004;200:957-65.
47: Tilburgs T, Scherjon SA, van der Mast BJ, Haasnoot GW, Versteeg-V D Voort-Maarschalk M, Roelen DL, van Rood JJ, Claas FH. Fetal-maternal HLA-C mismatch is associated with decidual T cell activation and induction of functional T regulatory cells. J Reprod Immunol. 2009; 82:148-57.
48: Kahn DA, Baltimore D. Pregnancy induces a fetal antigen-specific maternal T regulatory cell response that contributes to tolerance. Proc Natl Acad Sci U S A. 2010 18;107:9299-304.
49: King, A., D.S.J. Allan, J.M. Bowen, S.J. Powis, S. Joseph, S.Verma, S.E. Hiby, A.J. McMichael, Y.W. Loke, and V.M.Braud. 2000. HLA-E is expressed on trophoblast and interacts with CD94/NKG2 receptors on decidual NK cells. Eur.J. Immunol. 30:1623-1631.
50: Sato K, Fujita S. Dendritic cells: nature and classification. Allergol Int. 2007;56:183-91.
51:Maldonado RA, von Andrian UH. How tolerogenic dendritic cells induce regulatory T cells. Adv Immunol. 2010;108:111-65.
52: Haniffa M, Collin M, Ginhoux F. Ontogeny and functional specialization of dendritic cells in human and mouse. Adv Immunol. 2013;120:1-49.
53: Dalod M, Chelbi R, Malissen B, Lawrence T. Dendritic cell maturation: functional specialization through signaling specificity and transcriptional programming. EMBO J. 2014;33:1104-16.
54: Linscheid C, Petroff MG. Minor histocompatibility antigens and the maternal immune response to the fetus during pregnancy. Am J Reprod Immunol. 2013;69:304-14.
55: Hutter H, Dohr G. HLA expression on immature and mature human germ cells. J Reprod Immunol. 1998;38:101-22.
56: Guerin LR, Moldenhauer LM, Prins JR, Bromfield JJ, Hayball JD, Robertson SA. Seminal fluid regulates accumulation of FOXP3+ regulatory T cells in the preimplantation mouse uterus through expanding the FOXP3+ cell pool and CCL19-mediated recruitment. Biol Reprod. 2011;85:397-408.
57: Horie A, Fujiwara H, Sato Y, Suginami K, Matsumoto H, Maruyama M, Konishi I, Hattori A.Laeverin/aminopeptidase Q induces trophoblast invasion during human early placentation. Hum Reprod. 2012;27:1267-76.
58: Wegmann TG, Lin H, Guilbert L, Mosmann TR. Bidirectional cytokine interactions in the maternal-fetal relationship: is successful pregnancy a TH2 phenomenon? Immunol Today. 1993;14:353-6.
59: Raghupathy R. Th1-type immunity is incompatible with successful pregnancy. Immunol Today. 1997;18:478-82.
60: Piccinni MP, Beloni L, Livi C, Maggi E, Scarselli G, Romagnani S. Defective production of both leukemia inhibitory factor and type 2 T-helper cytokines by decidual T cells in unexplained recurrent abortions. Nat Med. 1998;4:1020-4.
61: Ng SC, Gilman-Sachs A, Thaker P, Beaman KD, Beer AE, Kwak-Kim J. Expression of intracellular Th1 and Th2 cytokines in women with recurrent spontaneous abortion, implantation failures after IVF/ET or normal pregnancy. Am J Reprod Immunol. 002;48:77-86.
62: Kwak-Kim JY, Chung-Bang HS, Ng SC, Ntrivalas EI, Mangubat CP, Beaman KD, Beer AE, Gilman-Sachs A. Increased T helper 1 cytokine responses by circulating T cells are present in women with recurrent pregnancy losses and in infertile women with multiple implantation failures after IVF. Hum Reprod. 2003;18:767-73.
63: Saito S, Nakashima A, Shima T, Ito M. Th1/Th2/Th17 and regulatory T-cell paradigm in pregnancy. Am J Reprod Immunol. 2010;63:601-10.
64: Ledee N, Petitbarat M, Chevrier L, Vitoux D, Vezmar K, Rahmati M, Dubanchet S, Gahery H, Bensussan A, Chaouat G. The Uterine Immune Profile May Help Women with Repeated Unexplained Embryo Implantation Failure After In Vitro Fertilization. Am J Reprod Immunol. 2016;75:388-401.
65: Mandelboim O, Porgador A. NKp46. Int J Biochem Cell Biol. 2001;33:1147-50.
66: Fukui A, Ntrivalas E, Gilman-Sachs A, Kwak-Kim J, Lee SK, Levine R, Beaman K. Expression of natural cytotoxicity receptors and a2V-ATPase on peripheral blood NK cell subsets in women with recurrent spontaneous abortions and implantation failures. Am J Reprod Immunol. 2006;56:312-20.
67: Zhang Y, Zhao A, Wang X, Shi G, Jin H, Lin Q. Expressions of natural cytotoxicity receptors and NKG2D on decidual natural killer cells in patients having spontaneous abortions. Fertil Steril. 2008;90:1931-7.
68: Shemesh A, Tirosh D, Sheiner E, Tirosh NB, Brusilovsky M, Segev R, Rosental B, Porgador A. First Trimester Pregnancy Loss and the Expression of Alternatively Spliced NKp30 Isoforms in Maternal Blood and Placental Tissue. Front Immunol. 2015;6:189
69: Yokota M, Fukui A, Funamizu A, Nakamura R, Kamoi M, Fuchinoue K, Sasaki Y, Fukuhara R, Mizunuma H.Role of NKp46 expression in cytokine production by CD56-positive NK cells in the peripheral blood and the uterine endometrium. Am J Reprod Immunol. 2013;69:202-11.
70: Fukui A, Kamoi M, Funamizu A, Fuchinoue K, Chiba H, Yokota M, Fukuhara R, Mizunuma H. NK cell abnormality and its treatment in women with reproductive failures such as recurrent pregnancy loss, implantation failures, preeclampsia, and pelvic endometriosis. Reprod Med Biol. 2015;14:151-157.
71: Gamen S, Hanson DA, Kaspar A, Naval J, Krensky AM, Anel A. Granulysin-induced apoptosis. I. Involvement of at least two distinct pathways. J Immunol. 1998;161:1758-64.
72: Clayberger C, Finn MW, Wang T, Saini R, Wilson C, Barr VA, Sabatino M, Castiello L, Stroncek D, Krensky AM. 15 kDa granulysin causes differentiation of monocytes to dendritic cells but lacks cytotoxic activity. J Immunol. 2012;188:6119-26.
73: Kino T, Hatanaka H, Hashimoto M, Nishiyama M, Goto T, Okuhara M, Kohsaka M, Aoki H, Imanaka H. FK-506, a novel immunosuppressant isolated from a Streptomyces. I. Fermentation, isolation, and physico-chemical and biological characteristics. J Antibiot (Tokyo). 1987;40:1249-55
74: Todo S, Fung JJ, Starzl TE, Tzakis A, Demetris AJ, Kormos R, Jain A, Alessiani M, Takaya S, Shapiro R. Liver, kidney, and thoracic organ transplantation under FK 506. Ann Surg. 1990;212:295-305;discussion 306-7.
75: McCaffrey PG, Luo C, Kerppola TK, Jain J, Badalian TM, Ho AM, Burgeon E, Lane WS, Lambert JN, Curran T, et al. Isolation of the cyclosporin-sensitive T cell transcription factor NFATp. Science. 1993;262:750-4.
76: Shibasaki F, Price ER, Milan D, McKeon F. Role of kinases and the phosphatase calcineurin in the nuclear shuttling of transcription factor NF-AT4. Nature. 1996;382:370-3.
77: Mayer AD, Dmitrewski J, Squifflet JP, Besse T, Grabensee B, Klein B, Eigler FW, Heemann U, Pichlmayr R, Behrend M, Vanrenterghem Y, Donck J, van Hooff J, Christiaans M, Morales JM, Andres A, Johnson RW, Short C, Buchholz B, Rehmert N, Land W, Schleibner S, Forsythe JL, Talbot D, Pohanka E, et al. Multicenter randomized trial comparing tacrolimus (FK506) and cyclosporine in the prevention of renal allograft rejection: a report of the European Tacrolimus Multicenter Renal Study Group. Transplantation. 1997;64:436-43.
78: Pirsch JD, Miller J, Deierhoi MH, Vincenti F, Filo RS. A comparison of tacrolimus (FK506) and cyclosporine for immunosuppression after cadaveric renal transplantation. FK506 Kidney Transplant Study Group. Transplantation. 1997;63:977-83.
79: Northrop JP, Ho SN, Chen L, Thomas DJ, Timmerman LA, Nolan GP, Admon A, Crabtree GR.NF-AT components define a family of transcription factors targeted in T-cell activation. Nature. 1994;369:497-502.
80: Masuda ES, Imamura R, Amasaki Y, Arai K, Arai N. Signalling into the T-cell nucleus: NFAT regulation. Cell Signal. 1998;10:599-611.
81: Oh-hora M, Rao A. Calcium signaling in lymphocytes. Curr Opin Immunol. 2008;20:250-8.
82: Smith-Garvin JE, Koretzky GA, Jordan MS. T cell activation. Annu Rev Immunol. 2009;27:591-619.
83: Jun O. Liu. Calmodulin-dependent phosphatase, kinases, and transcriptional corepressors involved in T-cell activation. Immunol Rev. 2009; 228: 184-198.
84: Plosker GL, Foster RH. Tacrolimus: a further update of its pharmacology and therapeutic use in the management of organ transplantation. Drugs. 2000;59:323-89.
85: Fung JJ. Tacrolimus and transplantation: a decade in review. Transplantation. 2004;77:S41-3.
86: Demmers MW, Korevaar SS, Betjes MG, Weimar W, Rowshani AT, Baan CC. Limited efficacy of immunosuppressive drugs on CD8+ T cell-mediated and natural killer cell-mediated lysis of human renal tubular epithelial cells. Transplantation. 2014;97:1110-8.
87: Wallin EF, Hill DL, Linterman MA, Wood KJ. The Calcineurin Inhibitor Tacrolimus Specifically Suppresses Human T Follicular Helper Cells. Front Immunol. 2018;9:1184.
88: Woltman AM, de Fijter JW, Kamerling SW, Paul LC, Daha MR, van Kooten C. The effect of calcineurin inhibitors and corticosteroids on the differentiation of human dendritic cells. Eur J Immunol. 2000;30:1807-12.
89: Cos J, Villalba T, Parra R, Gallardo D, Bilbao I, Margarit C, Massuet L. FK506 in the maturation of dendritic cells. Haematologica. 2002;87:679-87; discussion 687.
90: Ren Y, Yang Y, Yang J, Xie R, Fan H. Tolerogenic dendritic cells modified by tacrolimus suppress CD4(+) T-cell proliferation and inhibit collagen-induced arthritis in mice. Int Immunopharmacol. 2014;21:247-54.
91: Li H, Shi B. Tolerogenic dendritic cells and their applications in transplantation. Cell Mol Immunol. 2015;12:24-30.
92: Ghartey-Kwansah G, Li Z, Feng R, Wang L, Zhou X, Chen FZ, Xu MM, Jones O, Mu Y, Chen S, Bryant J, Isaacs WB, Ma J, Xu X. Comparative analysis of FKBP family protein: evaluation, structure, and function in mammals and Drosophila melanogaster. BMC Dev Biol. 2018 Mar 27;18:7. doi: 10.1186/s12861-018-0167-3.
93: Hutchinson IV, Bagnall W, Bryce P, Pufong B, Geraghty P, Brogan I. Differences in the mode of action of cyclosporine and FK 506. Transplant Proc. 1998;30:959-60.
94: Davies TH, Ning YM, Sanchez ER. Differential control of glucocorticoid receptor hormone-binding function by tetratricopeptide repeat (TPR) proteins and the immunosuppressive ligand FK506. Biochemistry. 2005;44:2030-8.
95: Takeuchi H, Iwamoto H, Nakamura Y, Hirano T, Konno O, Kihara Y, Chiba N, Yokoyama T, Takano K, Toraishi T, Okuyama K, Ikeda C, Tanaka S, Onda K, Soga A, Kikuchi Y, Kawaguchi T, Kawachi S, Unezaki S, Shimazu M. Synergistic Effects of Calcineurin Inhibitors and Steroids on Steroid Sensitivity of Peripheral Blood Mononuclear Cells. Cell Med. 2014;7:51-7.
96: Jain A, Venkataramanan R, Fung JJ, Gartner JC, Lever J, Balan V, Warty V, Starzl TE. Pregnancy after liver transplantation under tacrolimus. Transplantation. 1997;64:559-65.
97: Kainz A, Harabacz I, Cowlrick IS, Gadgil SD, Hagiwara D. Review of the course and outcome of 100 pregnancies in 84 women treated with tacrolimus. Transplantation. 2000;70:1718-21.
98: Nagy S, Bush MC, Berkowitz R, Fishbein TM, Gomez-Lobo V. Pregnancy outcome in liver transplant recipients. Obstet Gynecol. 2003;102:121-8.
99: Jain AB, Reyes J, Marcos A, Mazariegos G, Eghtesad B, Fontes PA, Cacciarelli TV, Marsh JW, de Vera ME, Rafail A, Starzl TE, Fung JJ. Pregnancy after liver transplantation with tacrolimus immunosuppression: a single center's experience update at 13 years. Transplantation. 2003;76:827-32.
100: Jain AB, Shapiro R, Scantlebury VP, Potdar S, Jordan ML, Flohr J, Marcos A, Fung JJ. Pregnancy after kidney and kidney-pancreas transplantation under tacrolimus: a single center's experience. Transplantation. 2004;77:897-902.
101: Garcia-Donaire JA, Acevedo M, Gutierrez MJ, Manzanera MJ, Oliva E, Gutierrez E, Andres A, Morales JM. Tacrolimus as basic immunosuppression in pregnancy after renal transplantation. A single-center experience. Transplant Proc. 2005;37:3754-5.
102: Christopher V, Al-Chalabi T, Richardson PD, Muiesan P, Rela M, Heaton ND, O'Grady JG, Heneghan MA. Pregnancy outcome after liver transplantation: a single-center experience of 71 pregnancies in 45 recipients. Liver Transpl. 2006;12:1138-43.
103: Oliveira LG, Sass N, Sato JL, Ozaki KS, Medina Pestana JO. Pregnancy after renal transplantation--a five-yr single-center experience. Clin Transplant. 2007;21:301-4.
104: Perales-Puchalt A, Vila Vives JM, Lopez Montes J, Diago Almela VJ, Perales A. Pregnancy outcomes after kidney transplantation-immunosuppressive therapy comparison. J Matern Fetal Neonatal Med. 2012;25:1363-6.
105: Webster P, Wardle A, Bramham K, Webster L, Nelson-Piercy C, Lightstone L. Tacrolimus is an effective treatment for lupus nephritis in pregnancy. Lupus. 2014;23:1192-6.
106: Nevers W, Pupco A, Koren G, Bozzo P. Safety of tacrolimus in pregnancy. Can Fam Physician. 2014;60:905-6.
107: Coscia LA, Constantinescu S, Davison JM, Moritz MJ, Armenti VT. Immunosuppressive drugs and fetal outcome. Best Pract Res Clin Obstet Gynaecol. 2014;28:1174-87.
108: Mohamed-Ahmed O, Nelson-Piercy C, Bramham K, Gao H, Kurinczuk JJ, Brocklehurst P, Pregnancy outcomes in liver and cardiothoracic transplant recipients: a UK national cohort study. PLoS One. 2014;9:e89151.
109: Coscia LA, Constantinescu S, Armenti DP, Moritz MJ. The 25th Anniversary of the National Transplantation Pregnancy Registry. Clin Transpl. 2015;31:57-68.
110: Piccoli GB, Cabiddu G, Attini R, Gerbino M, Todeschini P, Perrino ML, Manzione AM, Piredda GB, Gnappi E, Caputo F, Montagnino G, Bellizzi V, Di Loreto P, Martino F, Montanaro D, Rossini M, Castellino S, Biolcati M, Fassio F, Loi V, Parisi S, Versino E, Pani A, Todros T; Italian Study Group on Kidney and Pregnancy of the Italian Society of Nephrology. Pregnancy outcomes after kidney graft in Italy: are the changes over time the result of different therapies or of different policies? A nationwide survey (1978-2013). Nephrol Dial Transplant. 2016;31:1957-65.
111: Kanzaki Y, Kondoh E, Kawasaki K, Mogami H, Chigusa Y, Konishi I. Pregnancy outcomes in liver transplant recipients: A 15-year single-center experience. J Obstet Gynaecol Res. 2016;42:1476-82.
112: Tranguch S, Cheung-Flynn J, Daikoku T, Prapapanich V, Cox MB, Xie H, Wang H, Das SK, Smith DF, Dey SK. Cochaperone immunophilin FKBP52 is critical to uterine receptivity for embryo implantation. Proc Natl Acad Sci U S A. 2005;102:14326-31.
113: Tranguch S, Wang H, Daikoku T, Xie H, Smith DF, Dey SK. FKBP52 deficiency-conferred uterine progesterone resistance is genetic background and pregnancy stage specific.J Clin Invest. 2007;117:1824-34.
114: Nakagawa K, Kwak-Kim J, Ota K, Kuroda K, Hisano M, Sugiyama R, Yamaguchi K. Immunosuppression with tacrolimus improved reproductive outcome of women with repeated implantation failure and elevated peripheral blood TH1/TH2 cell ratios. Am J Reprod Immunol. 2015;73:353-61.
115: Nakagawa K, Kwak-Kim J, Kuroda K, Sugiyama R, Yamaguchi K. Immunosuppressive treatment using tacrolimus promotes pregnancy outcome in infertile women with repeated implantation failures. Am J Reprod Immunol. 2017;78. doi: 10.1111/aji.12682. Epub 2017 May 3.
116: Nakagawa K, Kuroda K, Sugiyama R, Yamaguchi K. After 12 consecutive miscarriages, a patient received immunosuppressive treatment and delivered an intact baby. Reprod Med Biol. 2017;16:297-301.
117: Moffett A, Regan L, Braude P. Natural killer cells, miscarriage, and infertility. BMJ. 2004;329:1283-5.
118: Park DW, Lee HJ, Park CW, Hong SR, Kwak-Kim J, Yang KM. Peripheral blood NK cells reflect changes in decidual NK cells in women with recurrent miscarriages. Am J Reprod Immunol. 2010;63:173-80.

1a: American College of Obstetricians and Gynecologists. ACOG Practice Bulletin No.75: Management of Alloimmunization. Obstet Gynecol 2006;108: 457-464.
2a: Moise KJ Jr. Management of rhesus alloimmunization in pregnancy. Obstet Gynecol 2002;100:600-611.
3a: Wylie BJ, D'Alton ME. Fetomaternal hemorrhage. Obstet Gynecol 2010;115:1039-1051.
4a: Pollack W, Ascari WQ, Kochesky RJ, O'Connor RR, Ho TY, Tripodi D. Studies on Rh prophylaxis. 1. Relationship between doses of anti-Rh and size of antigenic stimulus. Transfusion 1971;11:333-339.
5a: Mari G, Deter RL, Carpenter RL, Rahman F, Zimmerman R, Moise KJ Jr, Dorman KF, Ludomirsky A, Gonzalez R, Gomez R, Oz U, Detti L, Copel JA, Bahado-Singh R, Berry S, Martinez-Poyer J, Blackwell SC. Noninvasive diagnosis by Doppler ultrasonography of fetal anemia due to maternal red-cell alloimmunization. Collaborative Group for Doppler Assessment of the Blood Velocity in Anemic Fetuses. N Engl J Med 2000;342:9-14.
6a: Kwak-Kim J, Chung-Bang HS, Ng SC, Ntrivalas EI, Mangubat CP, Beaman KD, Beer AE, Gilman-Sachs A: Hum Reprod 2003; 18: 767-773.
7a: Schjenken JE, Tolosa JM, Paul JW, Clifton VL, Smith R: Croatia, INTECH, 2012, pp211-242.
8a: Aito S, Nakashima A, Shima T, Ito M: Am J Reprod Immunol 2010; 63: 601-610.
9a: Ng SC, Gilman-Sachs A, Thakar P, Beaman KD, Beer AE, Kwak-Kim J: Am J Reprod Immunol 2002; 48: 77-86.
10a: Riley JK: Immunol Invest 2008; 37: 395-426.
11a: Zilliacus H, Vartiainen E. Ottelin AM. Adult hemoglobin in the blood of very young human embryos. Nature 1962;193:386-387.
12a: Clayton EM Jr, Feldhaus WD, Whitacre FE. Fetal erythrocytes in the maternal circulation of pregnant women. Obstet Gynecol 1964;23:915-919.
13a: Nakagawa K, Kwak-Kim J, Ota K, Kuroda K, Hisano M, Sugiyama R, Yamaguchi K. Immunosuppression with Tacrolimus Improved Reproductive Outcome of Women with Repeated Implantation Failure and Elevated Peripheral Blood Th1/Th2 Cell Ratios. Am J Reprod Immunol. 2015; 73: 353-61.
14a: Nakagawa K, Kwak-Kim J, Kuroda K, Sugiyama R, Yamaguchi K. Immunosuppressive treatment using tacrolimus promotes pregnancy outcome in infertile women with repeated implantation failures. Am J Reprod Immunol. Sep;78(3). doi: 10.1111/aji.12682. Epub 2017 May 3.
15a: Nakagawa K, Kuroda K, Sugiyama R, Yamaguchi K. After 12 consecutive miscarriages, a patient received immunosuppressive treatment and delivered an intact baby. Reprod Med Biol. 2017;16:297-301.
16a: De la Camara C, Arrieta R, Gonzalez A, Iglesias E, Omenaca F. High-dose intravenous immunoglobulin as the sole prenatal treatment for severe Rh immunization. N Engl J Med 1988;318:519-520.
17a: Margulies M, Voto LS, Mathet E, Margulies M. High-dose intravenous IgG for the Treatment of severe rhesus alloimmunization. Vox Sang 1991;61:181-189.
18a: Ruma MS, Moise KJ Jr., Kim E, Murtha AP, Prutsman WJ, Hassan SS, Lubarsky SL. Combined plasmapheresis and intravenous immunoglobulin for the treatment of severe maternal red cell alloimmunization. Am J Obstet Gynecol 2007;196: 138e1-138e6.
19a: Isojima S, Hisano M, Suzuki T, Sago H, Murashima A and Yamaguchi K. Early plasmapheresis followed by high-dose -globulin treatment saved a severely Rho-incompatible pregnancy. Journal of Clinical Apheresis 2011; 26: 216-8.

1b: Sibai BM, Ramadan MK, Usta I, Salama M, Mercer BM, Friedman SA. Maternal morbidity and mortality in 442 pregnancies with hemolysis, elevated liver enzymes, and low platelets (HELLP syndrome) Am J Obstet Gynecol. 1993;169:1000-6.
2b: Sibai B, Dekker G, Kupferminc M. Pre-eclampsia. Lancet. 2005;365:785-99.
3b: Saito S, Sakai M. Th1/Th2 balance in preeclampsia. J Reprod Immunol. 2003;59:161-73.
4b: Y Sasaki, D Darmochwal-Kolarz, D Suzuki, M Sakai, M Ito, T Shima, and others. Proportion of peripheral blood and decidual CD4+ CD25bright regulatory T cells in pre-eclampsia. Clin Exp Immunol. 2007; 149: 139-145.
5b: Szarka A, Rigo J, Lazar L, Beko G, and Molvarec A. Circulating cytokines, chemokines and adhesion molecules in normal pregnancy and preeclampsia determined by multiplex suspension array. BMC Immunol. 2010; 11: 59.
6b: Powe CE, Levine RJ, Karumanchi SA. Preeclampsia, a disease of the maternal endothelium: the role of antiangiogenic factors and implications for later cardiovascular disease. Circulation. 2011;123:2856-69.
7b: Darmochwal-Kolarz D, Kludka-Sternik M, Tabarkiewicz J, Kolarz B, Rolinski J, Leszczynska-Gorzelak B, and others. The predominance of Th17 lymphocytes and decreased number and function of Treg cells in preeclampsia. J Reprod Immunol. 2012;93:75-81.
8b: Chaiworapongsa T, Chaemsaithong P, Yeo L, Romero R. Pre-eclampsia part 1: current understanding of its pathophysiology. Nat Rev Nephrol. 2014;10:466-80.
9b: Mol BWJ, Roberts CT, Thangaratinam S, Magee LA, de Groot CJM, Hofmeyr GJ. Pre-eclampsia. Lancet. 2016;387:999-1011.
10b: Salazar Garcia MD, Mobley Y, Henson J, Davies M, Skariah A, Dambaeva S, and others. Early pregnancy immune biomarkers in peripheral blood may predict preeclampsia. J Reprod Immunol. 2018;125:25-31.
11b: Yamaguchi K. Tacrolimus treatment for infertility related to maternal-fetal immune interactions. Am J Reprod Immunol. 2019;81:e13097.
12b: Nakagawa K, Kwak-Kim J, Ota K, Kuroda K, Hisano M, Sugiyama R, Yamaguchi K. Immunosuppression with Tacrolimus Improved Reproductive Outcome of Women with Repeated Implantation Failure and Elevated Peripheral Blood Th1/Th2 Cell Ratios. Am J Reprod Immunol. 2015; 73: 353-61.
13b: Rolnik DL, Wright D, Poon LC, O'Gorman N, Syngelaki A, de Paco Matallana C, and others. Aspirin versus Placebo in Pregnancies at High Risk for Preterm Preeclampsia. N Engl J Med. 2017;377:613-622.

## Claims

1. A medicine for inhibiting over-activated immunity before pregnancy or over-reactive immunity after pregnancy and restoring a normal immune state,
the medicine comprising, as an active ingredient, a compound selected from the group consisting of:
(i) a compound represented by Formula (I) or a pharmaceutically acceptable salt thereof, 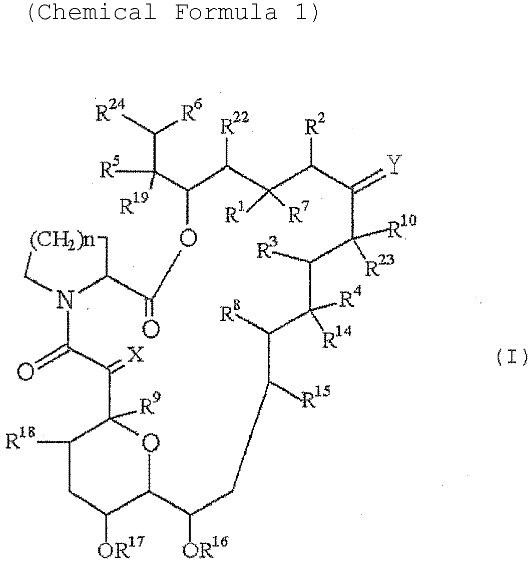 wherein each of the adjacent pairs of R¹ with R², R³ with R⁴, and R⁵ with R⁶ independently
(a) represents two adjacent hydrogen atoms, or R² may be an alkyl group, or
(b) may form another bond between the carbon atoms to which the pair members are respectively bonded;
R⁷ represents a hydrogen atom, a hydroxy group, or a protected hydroxy group, or may be bonded to R¹ and together represent an oxo group;
R⁸ and R⁹ each independently represent a hydrogen atom or a hydroxy group;
R¹⁰ represents a hydrogen atom, an alkyl group, an alkyl group substituted with one or more hydroxy groups, an alkenyl group, an alkenyl group substituted with one or more hydroxy groups, or an alkyl group substituted with an oxo group;
X represents an oxo group, (a hydrogen atom, a hydroxy group), (a hydrogen atom, a hydrogen atom), or a group represented by formula: -CH₂O-;
Y represents an oxo group, (a hydrogen atom, a hydroxy group), (a hydrogen atom, a hydrogen atom), or a group represented by formula: N-NR¹¹R¹² or formula: N-OR¹³;
R¹¹ and R¹² each independently represent a hydrogen atom, an alkyl group, an aryl group, or a tosyl group;
R¹³, R¹⁴, R¹⁵, R¹⁶, R¹⁷, R¹⁸, R¹⁹, R²², and R²³ each independently represent a hydrogen atom or an alkyl group;
R²⁴ represents a ring which can include one or more heteroatoms and may be substituted as desired;
n represents 1 or 2; and
in addition to the meanings described above, Y, R¹⁰, and R²³ may also be bonded together with the carbon atoms to which Y, R¹⁰, and R²³ are bonded, and represent a heterocyclic group having a saturated or unsaturated, 5-membered or 6-membered ring and containing one or more heteroatoms selected from a nitrogen atom, a sulfur atom, and an oxygen atom, while the heterocyclic group may be substituted with one or more groups selected from an alkyl group, a hydroxy group, an alkyloxy group, a benzyl group, a group represented by formula: -CH₂Se(C₆H₅), and an alkyl group substituted with one or more hydroxy groups,
(ii) a cyclosporine, and
(iii) rapamycin or a derivative thereof.

2. A medicine for inducing enhancement of the action of a steroid hormone through activation and nuclear translocation of a steroid hormone receptor,
the medicine comprising, as an active ingredient, a compound selected from the group consisting of:
(i) a compound represented by Formula (I) or a pharmaceutically acceptable salt thereof,
(ii) a cyclosporine, and
(iii) rapamycin or a derivative thereof.

3. A medicine for suppressing the activity of natural killer cells and natural killer T cells or macrophages, which are represented by natural immunity that has a possibility of being activated by a fertilized ovum or a fetal component in the uterus,
the medicine comprising, as an active ingredient, a compound selected from the group consisting of:
(i) a compound represented by Formula (I) or a pharmaceutically acceptable salt thereof,
(ii) a cyclosporine, and
(iii) rapamycin or a derivative thereof.

4. A medicine for inhibiting the activity of antigen-presenting cells presenting antigens of a fertilized ovum or a fetal component, cytotoxic T cells, or T cells producing an intercellular transmitter, all of which have a possibility of directly or indirectly attacking a fertilized ovum or a fetus by acquired immunity in the uterus,
the medicine comprising, as an active ingredient, a compound selected from the group consisting of:
(i) a compound represented by Formula (I) or a pharmaceutically acceptable salt thereof,
(ii) a cyclosporine, and
(iii) rapamycin or a derivative thereof.

5. A medicine for inducing antigen-presenting cells necessary for subjecting a fertilized ovum or a fetus to immunological tolerance or for inducing differentiation from undifferentiated dendritic cells to tolerant dendritic cells,
the medicine comprising, as an active ingredient, a compound selected from the group consisting of:
(i) a compound represented by Formula (I) or a pharmaceutically acceptable salt thereof,
(ii) a cyclosporine, and
(iii) rapamycin or a derivative thereof.

6. A medicine for suppressing humoral immunity responses to fetal components including a human leukocyte antigen (HLA), that is, production of a fetus-specific antibody,
the medicine comprising, as an active ingredient, a compound selected from the group consisting of:
(i) a compound represented by Formula (I) or a pharmaceutically acceptable salt thereof,
(ii) a cyclosporine, and
(iii) rapamycin or a derivative thereof.

7. A medicine for suppressing production of a pathogenic antibody in the mother's body, which brings about problems in the continuation of pregnancy,
the medicine comprising, as an active ingredient, a compound selected from the group consisting of:
(i) a compound represented by Formula (I) or a pharmaceutically acceptable salt thereof,
(ii) a cyclosporine, and
(iii) rapamycin or a derivative thereof.

8. A medicine for promoting suppression of activation of rejection immunity of the mother's body to the fetus and/or tolerance to the fetus,
the medicine comprising, as an active ingredient, a compound selected from the group consisting of:
(i) a compound represented by Formula (I) or a pharmaceutically acceptable salt thereof,
(ii) a cyclosporine, and
(iii) rapamycin or a derivative thereof.

9. A medicine for preventing the onset or delay the onset of pregnancy-induced hypertension syndrome and/or a disease associated with pregnancy-induced hypertension syndrome,
the medicine comprising, as an active ingredient, a compound selected from the group consisting of:
(i) a compound represented by Formula (I) or a pharmaceutically acceptable salt thereof,
(ii) a cyclosporine, and
(iii) rapamycin or a derivative thereof.

10. The medicine according to claim 9, wherein the disease associated with pregnancy-induced hypertension syndrome is HELLP syndrome.

11. The medicine according to claim 9, wherein the disease associated with pregnancy-induced hypertension syndrome is eclampsia.

12. The medicine according to any one of claims 1 to 11, wherein the active ingredient is a compound of Formula (I) or a pharmaceutically acceptable salt thereof, and the compound of Formula (I) is tacrolimus or a pharmaceutically acceptable salt thereof.
